(19) Europäisches Patentamt
European Patent Office
Office européen des brevets

(11) EP 4 247 256 B1

(12) EUROPEAN PATENT SPECIFICATION

(45) Date of publication and mention of the grant of the patent:
**03.09.2025 Bulletin 2025/36**

(21) Application number: **21824718.7**

(22) Date of filing: **17.11.2021**

(51) International Patent Classification (IPC):
***A61B 5/1486*** (2006.01) ***A61B 5/145*** (2006.01)

(52) Cooperative Patent Classification (CPC):
**A61B 5/1486; A61B 5/14532;** A61B 5/14865; A61B 5/4839; A61B 5/6848; A61B 5/686

(86) International application number:
**PCT/US2021/059691**

(87) International publication number:
**WO 2022/109008 (27.05.2022 Gazette 2022/21)**

(54) **GLUCOSE SENSORS**

GLUKOSESENSOREN

CAPTEURS DE GLUCOSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.11.2020 US 202063115474 P**

(43) Date of publication of application:
**27.09.2023 Bulletin 2023/39**

(73) Proprietor: **Willow Laboratories, Inc.**
**Irvine, CA 92618 (US)**

(72) Inventors:
- **KRISHNAMANI, Venkatramanan**
  **Irvine, California 92618 (US)**
- **PIRBADIAN, Sahand**
  **Irvine, California 92618 (US)**
- **PAULEY, Kevin Hughes**
  **Irvine, California 92618 (US)**
- **BALASHOV, Sergei Petrovich**
  **Irvine, California 92618 (US)**
- **DEVADOSS, Anando**
  **Irvine, California 92618 (US)**

(74) Representative: **Michalski Hüttermann & Partner Patentanwälte mbB**
**Kaistraße 16A**
**40221 Düsseldorf (DE)**

(56) References cited:
**US-A- 5 431 160** **US-A1- 2017 238 851**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 247 256 B1

## Description

BACKGROUND

The present disclosure relates to physiological monitoring devices. More specifically, this disclosure relates to glucose monitoring devices and methods of making the same.

BACKGROUND

Monitoring of blood glucose concentration levels has long been critical to the management and care of diabetes mellitus. Current blood glucose monitors involve a chemical reaction between blood or serum and a test strip, requiring an invasive extraction of blood via a lancet or pinprick. Small handheld monitors have been developed to enable a patient to perform this procedure anywhere, at any time. But the inconvenience of this procedure - specifically the blood extraction, the pain associated with the procedure and the use and disposition of lancets and test strips - has led to a low level of compliance. As such, it is desirable to continuously monitor the concentration of glucose level in the human body.

A first generation of electrochemical continuous glucose monitoring (CGM) sensor was developed based on Clark-type amperometric detection. Such CGM sensor measures the current generated by the electrochemical oxidation of hydrogen peroxide ($H_2O_2$) at the surface of either an activated platinum or a platinum/iridium (90:10 Pt:Ir) electrode. Hydrogen peroxide ($H_2O_2$) is a byproduct of oxidation (loss of electrons) of glucose by the enzyme glucose oxidase (GOx). Each molecule of glucose oxidized by GOx generates one molecule of $H_2O_2$ as a byproduct, which acts as a proxy for measuring the glucose concentration. The $H_2O_2$ fraction that diffuses inward (towards the electrode) gets electrochemically oxidized into oxygen at the Pt surface thereby generating current to measure glucose concentration.

$$glucose + O_2 \xrightarrow{glucose\ oxidase} gluconolactone + H_2O_2$$

$$H_2O_2 \rightarrow O_2 + 2H^+ + 2e^-$$

However, some fraction of $H_2O_2$ lingers within the enzymatic GOx layer and other fraction of $H_2O_2$ diffuses outwards (to the outside). These fractions of $H_2O_2$ that are not electrochemically oxidized by the electrode renders irreversible oxidative damage to all the layers of the sensor. This oxidative damage slowly changes the sensitivity and efficacy of the sensor towards detecting glucose, leading to undesirable and uncorrectable drift in the sensor over its lifetime. Furthermore, the presence of various endogenous and exogenous species in the bodily fluid or serum that are redox active at the operating potential of the sensor may also interfere with accurate measurements of the glucose level.

More recent CGM technology uses semi-selective polymer membranes to achieve blocking of such interferences but also to equalize the concentrations of glucose and oxygen at the GOx enzyme layer. As a consequence, the membranes also block/reduce the diffusion of all molecules (based on size) making sensor engineering a complicated task. Furthermore, the temperature sensitivity of such glucose sensors remains a problem. Therefore, there remains a need to develop CGM sensors that are temperature independent within a wide temperature range as a result of physiological condition (e.g., human body temperature changes due to hypothermia and hyperpyrexia).

US 5 431 160 A describes a reusable, miniature, implantable electrochemical sensor, a method of making the same, and a powder therefor.

US 2017/238851 A1 describes sensor systems, transdermal analyte monitoring systems (TAMS), and methods of improving analyte detection.

SUMMARY

The invention is defined by the features of the independent claim.

In some embodiments of the first aspect of the glucose monitoring device described herein, the glucose oxidase in the enzymatic layer is present in a matrix comprising bovine serum albumin (BSA), poly(ortho-phenylenediamine) (PoPD), poly(meta-phenylenediamine) (PmPD), or poly(para-phenylenediamine) (PpPD), or combinations thereof. In some such embodiments, glucose oxidase may be co-electropolymerized with one or more of phenylene diamine (such as oPD, mPD, or pPD), or other electro-polymerizable monomers such as pyrrole, or aniline, or combinations thereof. In some other embodiments, the glucose oxidase is present in a hydrogel matrix. In some such embodiments, the hydrogel matrix comprises one or more materials selected from the group consisting of cellulose acetate, chitosan, poly(2-hydroxyethyl methacrylate)(pHEMA), polyethylene glycol diamine, 3,6,9-Trioxaundecanedioic acid, sodium citrate, polyvinyl alcohol and polyethylenimine(PEI), and combinations thereof. In some other embodiments, the hydrogel matrix comprises two or

more crosslinked materials described herein. In further embodiments, the hydrogel matrix may further comprise one or more polymeric materials that render the hydrogel matrix with a negative charge, for example, polymers bears cationic or anionic groups, or salts thereof. For example, the one or more polymeric materials may comprise poly(sodium 4-styrenesulfonate), poly(4-styrenesulfonic acid-co-maleic acid) sodium salt, poly(acrylic acid-co-maleic acid), or poly(vinylsulfonic acid) sodium salt, or combinations thereof. In some embodiments, the glucose oxidase is cross-linked. In some embodiments, the oxidized species of the reaction of glucose and oxygen in the enzymatic layer comprises hydrogen peroxide ($H_2O_2$) and gluconolactone.

In some embodiments of the first aspect of the glucose monitoring device described herein, the oxygen-replenishing layer comprises one or more enzymes selected from the group consisting of peroxidases, transferases, hydrolases, oxidases, kinases, superoxidases, phosphatases, pyrophosphatases, hydroxylases, dioxygenases, dehydrogenases, carboxylases, aminases, catalase, phosphohydrolases, diaminases, reductases, synthases, and caspases, and combinations thereof. In some further embodiments, the oxygen-replenishing layer comprises catalase to converts $H_2O_2$ to generate oxygen. In some embodiments, the oxygen-replenishing layer further comprises a glucokinase or a gluconate dehydrogenase to reduce or eliminate the accumulation of gluconolactone. In some embodiments, the oxygen-replenishing layer further comprises ascorbate peroxidase or ascorbate oxidase, which can reduce or eliminate interfering molecule ascorbic acid before it reaches to the working electrode. In some additional embodiments, either or both of the enzymatic layer and the oxygen-replenishing layer further comprises one or more oxygen binding proteins or oxygen binding globins, or combinations thereof. In some such embodiments, the oxygen binding protein comprises hemerythrin. In some such embodiments, the oxygen binding globin comprises myoglobin, hemoglobin, or a combination thereof.

In some embodiments of the first aspect of the glucose monitoring device described herein, the first permeability-selective layer comprises one or more polymers selected from the group consisting of a polyacetal, a polyolefin, a polyacrylic, a polycarbonate, a polystyrene, a polyester, a polyamide, polyamideimides, a polyarylate, a polyarylsulfone, a polyethersulfone, a polyphenylene sulfide, a polyvinyl chloride, a polyethylene oxide, a polysulfone, a polyimide, a polyetherimide, a polytetrafluoroethylene, a polyetherketone, a polyether etherketone, a polyether ketone ketone, a polybenzoxazole, a polyphthalide, a polyacetal, a polyanhydride, a polyvinyl ether, a polyvinyl thioether, a polyvinyl alcohol, a polyvinyl ketone, a polyvinyl halide, a polyvinyl nitrile, a polyvinyl ester, a polysulfonate, a polysulfide, a poly(allyl amine), a polythioester, a polysulfone, a polysulfonamide, a polyurea, a polyphosphazene, a polysilazane, a polyvinylchloride, a polyvinyl acetate, a humic acid, a cellulose acetate, a polythiophene, a polyphenylene diamine, a polypyrrole, a polynaphthalene a polyurethane, an ethylene propylene diene rubber, a polytetrafluoroethylene, a fluorinated ethylene propylene, a sulfonated tetrafluoroethylene based fluoropolymer-copolymer (e.g., Nafion™), a perfluoroalkoxyethylene, a polychlorotrifluoroethylene, a polyvinylidene fluoride, and a polysiloxane, and combinations thereof. In one embodiment, the first permeability-selective layer comprises Nafion™. In some further embodiments, the first permeability layer comprises or is a layer of poly(ortho-phenylenediamine) (PoPD), poly(meta-phenylenediamine) (PmPD), or poly(para-phenylenediamine) (PpPD), or combinations thereof. In some embodiments, the first permeability-selective layer is disposed between the enzymatic layer and the oxygen-replenishing layer. In some further embodiments, the first permeability-selective layer is in direct contact with one or both of the enzymatic layer and the oxygen-replenishing layer. In some other embodiments, the first permeability-selective layer is disposed between the oxygen-replenishing layer and the outer protective layer. In some further embodiments, the first permeability-selective layer is in direct contact with one or both of oxygen-replenishing layer and the outer protective layer.

In some embodiments of the first aspect of the glucose monitoring device described herein, the device further comprises a second permeability-selective layer for blocking the contact of one or more redox active species with the working electrode and/or the reference electrode. In some such embodiments, the second permeability-selective layer is disposed between the working electrode and the enzymatic layer. In some further embodiments, the second permeability-selective layer is in direct contact with one or both of the working electrode and the enzymatic layer. In some embodiments, the second permeability-selective layer comprises electropolymerized PoPD, electropolymerized PmPD, electropolymerized PpPD, diamino-naphthalene (DAN), amino naphthol, polypyrrole, polyaniline, cellulose acetate, or an ionic polymer (e.g., Nafion™), or combinations thereof. In some embodiments, the second permeability-selective layer has a thickness from about 1 nm to about 10 $\mu$m, from about 2 nm to about 1 $\mu$m, or from about 5 nm to about 500 nm. In some further embodiments, the second permeability-selective layer has a thickness of about 10 nm to about 300 nm. In some embodiments, the one or more redox active species comprises endogenous or exogenous compounds present in a mammal's bodily fluid, tissue fluid, or serum. In some further embodiments, the one or more redox active species comprises ascorbic acid, uric acid, or acetaminophen, or combinations thereof.

In some embodiments of the first aspect of the glucose monitoring device described herein, the second permeability-selective layer is disposed between the working electrode and the enzymatic layer, the enzymatic layer is disposed between the second permeability-selective layer and the first permeability-selective layer, the first permeability-selective layer is disposed between the enzymatic layer and the oxygen-replenishing layer, and the oxygen-replenishing layer is disposed between the first permeability-selective layer and the outer protective layer. In some further embodiments, the second permeability-selective layer is disposed between and in direct contact with either or both of the

working electrode and the enzymatic layer, the enzymatic layer is disposed between and in direct contact with either or both of the second permeability-selective layer and the first permeability-selective layer, the first permeability-selective layer is disposed between and in direct contact with either or both of the enzymatic layer and the oxygen-replenishing layer, and the oxygen-replenishing layer is disposed between and in direct contact with either or both the first permeability-selective layer and the outer protective layer.

In further embodiments of the first aspect of the glucose monitoring device described herein, any one or more of the first permeability-selective layer, the enzymatic layer, the oxygen-replenishing layer, the second permeability-selective layer, and the outer protective layer may further comprises one or more enzymes for reducing or eliminating the interference of one or more interfering molecules with the working electrode. In some such embodiments, the one or more interfering molecules comprise ascorbic acid, uric acid, or acetaminophen, hydroxyurea, cholesterol, creatinine, dopamine, ethylenediaminetetraacedic acid (EDTA), gentisic acid, heparin, or salicylic acid, or combinations thereof.

In some embodiments of the second aspect of the glucose monitoring device described herein, the glucose oxidase and the polymeric mediator are present in a hydrogel matrix. In some such embodiments, the hydrogel matrix comprises one or more materials selected from the group consisting of cellulose acetate, chitosan, poly(2-hydroxyethyl methacrylate)(pHEMA), polyethylene glycol diamine, 3,6,9-Trioxaundecanedioic acid, sodium citrate, polyvinyl alcohol and polyethylenimine(PEI), and combinations thereof. In some other embodiments, the hydrogel matrix comprises two or more crosslinked materials described herein. In further embodiments, the hydrogel matrix may further comprise one or more polymeric materials that render the hydrogel matrix with a negative charge, for example, polymers bears cationic or anionic groups, or salts thereof. For example, the one or more polymeric materials may comprise poly(sodium 4-styrenesulfonate), poly(4-styrenesulfonic acid-co-maleic acid) sodium salt, poly(acrylic acid-co-maleic acid), or poly(vinylsulfonic acid) sodium salt, or combinations thereof.

In some embodiments of the second aspect of the glucose monitoring device described herein, the polymeric mediator comprises a backbone material, one or more redox mediator moieties, wherein the one or more redox mediator moieties are attached to the backbone material optionally through one or more linkers. In some such embodiments, the backbone material comprises polyethylenimine (PEI), polyallylamine, cellulose, cellulose acetate, chitosan, poly(acrylic acid), poly(lactic acid), carbon nanofibers, carbon nanotubes, or metal nanofibers, or combinations thereof. In some such embodiments, the polymer mediator further comprises one or more functional groups for improving the water solubility of the polymeric mediator, wherein the one or more functional groups are attached to the backbone material optionally through one or more linkers. In some embodiments, the functional groups comprise cations or anions, or a combination thereof. For example, the functional groups may comprise $-SO_3^-$, $-PO_3^-$, $-NH_3^+$, or $-N(CH_3)_3^+$, or combinations thereof. In some embodiments, the one or more linkers comprises an alkylene linker, an heteroalkylene linker, a polyethylene glycol (PEG) linker, or combinations thereof. In some embodiments, wherein the one or more redox mediator moieties of the polymeric mediator comprise ferrocene or derivatives thereof, transition metal complexes, or organic molecules, or combinations thereof. In some such embodiments, the transition metal complex comprises iron-phenanthroline, a ruthenium complex, or a combination thereof. In some such embodiments, the organic molecule comprise viologens or quinones, or a combination thereof.

In some embodiments of the second aspect of the glucose monitoring device described herein, the enzymatic layer comprising the glucose oxidase and the polymeric mediator may further comprise a second enzyme. In some embodiments, the second enzyme is a peroxidase, for example, horseradish peroxidase. In another embodiment, the second enzyme is catalase.

In some embodiments of the second aspect of the glucose monitoring device described herein, the first permeability-selective layer comprises one or more polymers selected from the group consisting of a polyacetal, a polyolefin, a polyacrylic, a polycarbonate, a polystyrene, a polyester, a polyamide, polyamideimides, a polyarylate, a polyarylsulfone, a polyethersulfone, a polyphenylene sulfide, a polyvinyl chloride, a polyethylene oxide, a polysulfone, a polyimide, a polyetherimide, a polytetrafluoroethylene, a polyetherketone, a polyether etherketone, a polyether ketone ketone, a polybenzoxazole, a polyphthalide, a polyacetal, a polyanhydride, a polyvinyl ether, a polyvinyl thioether, a polyvinyl alcohol, a polyvinyl ketone, a polyvinyl halide, a polyvinyl nitrile, a polyvinyl ester, a polysulfonate, a polysulfide, a poly(allyl amine), a polythioester, a polysulfone, a polysulfonamide, a polyurea, a polyphosphazene, a polysilazane, a polyvinylchloride, a polyvinyl acetate, a humic acid, a cellulose acetate, a polythiophene, a polyphenylene diamine, a polypyrrole, a polynaphthalene a polyurethane, an ethylene propylene diene rubber, a polytetrafluoroethylene, a fluorinated ethylene propylene, a sulfonated tetrafluoroethylene based fluoropolymer-copolymer (e.g., Nafion™), a perfluoroalkoxyethylene, a polychlorotrifluoroethylene, a polyvinylidene fluoride, and a polysiloxane, and combinations thereof. In one embodiment, the first permeability-selective layer comprises Nafion™. In some further embodiments, the first permeability layer comprises or is a layer of poly(ortho-phenylenediamine) (PoPD), poly(meta-phenylenediamine) (PmPD), or poly(para-phenylenediamine) (PpPD), or combinations thereof. In some embodiments, the first permeability-selective layer is disposed between the enzymatic layer and the outer protective layer. In some further embodiments, the first permeability-selective layer is in direct contact with one or both of the enzymatic layer and the outer protective layer.

In some embodiments of the second aspect of the glucose monitoring device described herein, the device further

comprises a second permeability-selective layer for blocking the contact of one or more redox active species with the working electrode and/or the reference electrode. In some such embodiments, the second permeability-selective layer is disposed between the working electrode and the enzymatic layer. In some further embodiments, the second permeability-selective layer is in direct contact with one or both of the working electrode and the enzymatic layer. In some embodiments, the second permeability-selective layer comprises electropolymerized PoPD, electropolymerized PmPD, electropolymerized PpPD, diamino-naphthalene (DAN), amino naphthol, polypyrrole, polyaniline, cellulose acetate, or an ionic polymer (e.g., Nafion™), or combinations thereof. In some embodiments, the one or more redox active species comprises endogenous or exogenous compounds present in a mammal's bodily fluid, tissue fluid, or serum. In some further embodiments, the one or more redox active species comprises ascorbic acid, uric acid, or acetaminophen, or combinations thereof.

In some embodiments of the second aspect of the glucose monitoring device described herein, the second permeability-selective layer is disposed between the working electrode and the enzymatic layer, the enzymatic layer is disposed between the second permeability-selective layer and the first permeability-selective layer, the first permeability-selective layer is disposed between the enzymatic layer and the outer protective layer.

In some embodiments of any glucose monitoring devices described herein, the outer protective layer may be used for reducing or inhibiting protein adhesion. In some embodiments, the outer protective layer comprises a polymer, a hydrogel, or a combination thereof. In some such embodiments, the outer protective layer comprises polyvinyl alcohol (PVA), Nafion™, or a combination thereof. In one embodiment, the outer protective layer comprises crosslinked PVA. In some further embodiments, the outer protective layer further comprises an anti-inflammatory drug, an angiogenesis factor, or a combination thereof. In any embodiments of the outer protective layer, such layer is biocompatible.

In some embodiments of any glucose monitoring devices described herein, the working electrode and/or the reference electrode comprises one or more conductive materials, such as metals. In some further embodiments, the working electrode and/or the reference electrode comprises platinum, gold, silver, rhodium, iridium, carbon, graphite, silicon, or combinations or alloys thereof. In one embodiment, the working electrode comprises platinum (Pt). In another embodiment, the working electrode comprises both platinum and iridium. In one embodiment, the reference electrode comprises silver and silver chloride. In some embodiments, the device further comprises a counter electrode. The counter electrode may comprises one or more metals described herein. In one embodiment, the counter electrode comprises gold (Au).

Some additional aspect of the present disclosure relates to a method of implanting a glucose monitoring device to a subject in need thereof, comprising: contacting a glucose monitoring device described herein with an aqueous medium; and implanting the glucose monitor into a tissue of the subject. In some embodiments, the contacting of the glucose monitoring device with the aqueous medium leads to swelling of the enzymatic layer of the glucose monitoring device.

In any embodiments of the glucose monitoring device described herein, the device comprises or is a glucose sensor.

In any embodiments of the glucose monitoring devices described herein, the glucose monitoring device is an implantable continuous glucose monitoring (CGM) device. In some embodiments, the CGM device has an operating temperature range between about 35°C to about 41°C. In further embodiments, the glucose monitoring device does not comprise or require a temperature sensor, and/or does not comprise or require algorithmic correction for temperature related variability.

## BRIEF DESCRIPTION OF THE DRAWINGS

FIG. 1A is a schematic illustration of a typical first generation glucose sensor comprising three separate layers outside the working electrode.

FIG. 1B is an exemplary schematic illustration of a glucose monitoring device described herein comprising five separate layers outside the working electrode.

FIG. 1C is an exemplary schematic illustration of a glucose monitoring device described herein comprising three separate layers outside the working electrode, including a polymeric mediator in the enzymatic layer.

FIG. 2 is an exemplary schematic illustration of an glucose monitoring device described herein comprising five separate layers coating the working electrode, each layer comprises a specific type of material.

FIG. 3A is a diagram illustrating the general structure of a polymeric mediator based on a branched polymer backbone material.

FIG. 3B is a diagram illustrating the general structure of a polymeric mediator where one or two types of mediator molecules may be attached.

FIGs. 4A-4C illustrate an exemplary control system that may include a glucose monitoring device described herein.

FIG. 5 illustrates an exemplary disease management system that comprises a glucose monitoring device described

herein.

FIG. 6 illustrates an exemplary implementation of a disease management system described herein.

DETAILED DESCRIPTION

Aspects of the disclosure will now be set forth in detail with respect to the figures and various examples. One of skill in the art will appreciate, however, that other configurations of the devices and methods disclosed herein will still fall within the scope of this disclosure even if not described in the same detail. Aspects of various configurations discussed do not limit the scope of the disclosure herein, which is instead defined by the claims following this description.

Embodiments of the present disclosure relate to a temperature independent glucose monitoring device. In particular, the temperature sensitivity of typical glucose sensors have been addressed by using a unique combination of enzymes to cancel out glucose oxidases temperature correlated behavior. By making the glucose sensor independent of temperature eliminates the requirement for: (a) a temperature sensor within the CGM product; and (b) correction for temperature related errors and instabilities introduced to the system from algorithmic interpolation of temperature. The multi-enzyme containing glucose sensor described herein also specifically and reliably breaks down interfering molecules and thereby reduces or eliminates sensor hysteresis due to diffusion of molecules, and alleviates the diffusion challenge of by having multiple size exclusion based layers.

Definition

As used herein, common abbreviations are defined as follows:

| | |
|---|---|
| °C | Temperature in degrees Centigrade |
| CE | Counter electrode |
| CGM | Continuous glucose monitoring |
| DAN | Diamino naphthalene |
| GOx | Glucose oxidase |
| $H_2O_2$ | Hydrogen peroxide |
| oPD | ortho-Phenylenediamine |
| mPD | meta-Phenylenediamine |
| PBS | Phosphate buffered saline |
| pPD | para-Phenylenediamine |
| PmPD | Poly(meta-phenylenediamine) |
| PoPD | Poly(ortho-phenylenediamine) |
| PpPD | Poly(para-phenylenediamine) |
| PVA | Polyvinyl alcohol |
| RE | Reference electrode |
| WE | Working electrode |

Unless defined otherwise, all technical and scientific terms used herein have the same meaning as is commonly understood by one of ordinary skill in the art. The use of the term "including" as well as other forms, such as "include", "includes," and "included," is not limiting. The use of the term "having" as well as other forms, such as "have", "has," and "had," is not limiting. The terms "comprising," "including," "having," and the like are synonymous and are used inclusively, in an open-ended fashion, and do not exclude additional elements, features, acts, operations, and so forth. That is, the above terms are to be interpreted synonymously with the phrases "having at least" or "including at least." For example, when used in the context of a process, the term "comprising" means that the process includes at least the recited steps, but may include additional steps. When used in the context of a device, the term "comprising" means that the device includes at least the recited features or components, but may also include additional features or components. Also, the term "or" is used in its inclusive sense (and not in its exclusive sense) so that when used, for example, to connect a list of elements, the term "or" means one, some, or all of the elements in the list. Further, the term "each," as used herein, in addition to having its ordinary meaning, can mean any subset of a set of elements to which the term "each" is applied.

Conditional language, such as "can," "could," "might," or "may," unless specifically stated otherwise, or otherwise understood within the context as used, is generally intended to convey that certain embodiments include, while other embodiments do not include, certain features, elements, or steps. Thus, such conditional language is not generally intended to imply that features, elements, or steps are in any way required for one or more embodiments or that one or more embodiments necessarily include logic for deciding, with or without user input or prompting, whether these features, elements, or steps are included or are to be performed in any particular embodiment.

Conjunctive language such as the phrase "at least one of X, Y, and Z," unless specifically stated otherwise, is

otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than 10% of, within less than 5% of, within less than 1% of, within less than 0.1% of, and within less than 0.01% of the stated amount.

The term "and/or" as used herein has its broadest least limiting meaning which is the disclosure includes A alone, B alone, both A and B together, or A or B alternatively, but does not require both A and B or require one of A or one of B. As used herein, the phrase "at least one of" A, B, "and" C should be construed to mean a logical A or B or C, using a non-exclusive logical or.

The term "temperature independent" as used herein, means that the reading or measurement of the glucose level by the glucose monitoring device or the response of the glucose sensor is not affect or not substantially affected by the change of temperature. In other words, the sensor is insensitive the change of temperature (e.g., change of body temperature as a result of physiological conditions such as hypothermia and hyperpyrexia). In some embodiments, the temperature independent property of the glucose monitoring device is maintained within the operating temperature range of the device (e.g., from about 30°C to about 45°C, from about 33°C to about 43°C, from about 35°C to about 41°C, or from about 36°C to about 40°C. In some embodiments, the change of temperature (per °C) results in less than 5%, 4%, 3%, 2%, 1%, 0.5%, 0.1% or 0.01% change in the response of the sensor, or the measurement/reading provided by the device, when all the other parameters remain the same (e.g., the glucose concentration is constant).

Any methods disclosed herein need not be performed in the order recited. The methods disclosed herein include certain actions taken by a practitioner; however, they can also include any third-party instruction of those actions, either expressly or by implication.

## First Generation Glucose Sensors

FIG. 1A is a schematic view of an exemplary first generation glucose sensor. In this example, when implanted, glucose diffuses from the human body through the outer permeability selective layer 40 (first permeability selective layer or diffusion control layer) to the GOx enzymatic layer 30 where it gets catalyzed by the enzyme to generate $H_2O_2$ and gluconolactone. Glucose oxidase continuously catalyzes this reaction at a certain rate as long as the substrate (i.e., glucose) is available. A fraction of $H_2O_2$ diffuses inwards (through the interferents blocking layer 20 and towards the electrode 10), another fraction of $H_2O_2$ diffuses outwards (to the outside, through the outer permeability-selective layer 40, and there is also a fraction lingers within the enzymatic GOx layer. The $H_2O_2$ fraction that reaches the electrode gets electrochemically oxidized into oxygen at the Pt surface thereby generating current to measure glucose concentration. However, the other two fractions of $H_2O_2$ is lost to outside of the sensor by simple diffusion and renders irreversible oxidative damage to all the layers of the sensor, respectively. This oxidative damage slowly changes the sensitivity and efficacy of the sensor towards detecting glucose, leading to undesirable and uncorrectable drift in the sensor over its lifetime.

## Multi-Enzyme Containing Glucose Sensors

Some embodiments of the present disclosure relate to a glucose monitoring device comprising:

a reference electrode;
a working electrode, wherein the working electrode is disposed in the vicinity of the reference electrode;
an enzymatic layer comprising glucose oxidase, wherein the glucose oxidase is capable of catalyzing a reaction of glucose and oxygen to generate one or more oxidized species;
a first permeability-selective layer for reducing or blocking the diffusion of glucose to the enzymatic layer;
an oxygen-replenishing layer comprising one or more enzymes, wherein at least one enzyme in the oxygen-replenishing layer is capable of consuming at least one oxidized species from the enzymatic layer and generating oxygen; and
an outer protective layer;
wherein the enzymatic layer is in closer proximity to the working electrode than the oxygen-replenishing layer, and wherein the rate of reaction of the glucose oxidase in the enzymatic layer and the rate of reaction of the oxygen-generating enzyme in the oxygen-replenishing layer is substantially the same such that the glucose monitoring device is temperature independent within an operating temperature range.

*Oxygen-Replenishing Layer*

An embodiment of the improved multi-enzyme containing glucose sensor described herein is illustrated in FIG. 1B. In addition to the layers illustrated in FIG. 1A, it contains a catalase layer 50 (an embodiment of the oxygen-replenishing layer) and an outer permeability-selective layer 40 (an embodiment of the first permeability-selective layer or diffusion control layer) between the GOx layer 30 and the catalase layer 50. The oxygen-replenishing layer 50 (i.e. the catalase layer) continuously sequesters the highly reactive $H_2O_2$ from the GOx layer 30 and converts it into oxygen ($O_2$) and water.

$$2H_2O_2 \xrightarrow{catalase} O_2 + 2H_2O$$

The multi-enzyme containing glucose sensor described herein is independent to temperature variation. Human body temperature typically ranges from 35°C (hypothermia) to 41°C (hyperpyrexia). These temperatures would be the operating conditions of an implanted glucose sensor. The rate of an enzyme-catalyzed reaction increases as the temperature is raised. A ten degree rise in temperature will increase the activity of most enzymes by 50 to 100%. However, the specific activity change with temperature of each individual enzyme is varied. In the embodiment of the glucose sensor illustrated in FIG. 1B, both glucose oxidase and catalase activity increases when the temperature increases.

Overall, the fraction of $H_2O_2$ available at the electrode for electrochemical oxidation is a complex interplay of the rates of Gox and catalase, as well as the available substrate concentration - glucose for glucose oxidase and $H_2O_2$ for catalase. By optimizing the balance of enzyme loading of these two enzymes in the CGM sensor, given a constant glucose concentration is available to the sensor, the relative enzymatic activity change due to temperature variation will maintain a constant fraction of $H_2O_2$ available at the working electrode. In effect, making the sensor response independent of temperature.

The relationship between the products and substrate during catalysis by glucose oxidase is given by Michaelis-Menten kinetics:

$$\frac{d[H_2O_2]}{dt} = \frac{V_{max}^{GO}[G]}{K_M^{GO} + [G]}$$

Similarly, for catalase:

$$\frac{d[O_2]}{dt} = \frac{V_{max}^{CAT}[H_2O_2]}{K_M^{CAT} + [H_2O_2]}$$

With varying temperature both $V_{max}$ and $K_M$ of both enzymes change, but $V_{max}$ changes more rapidly than $K_M$. To achieve temperature independence, at a constant glucose concentration, the balance of catalase and glucose oxidase enzymes in the CGM sensor should be such that the increase in production of $H_2O_2$ due to temperature change proportionally increases the consumption of $H_2O_2$ by catalase such that the total amount of $H_2O_2$ reaching the electrode is maintained a constant. Since 2 molecules of $H_2O_2$ is converted into one molecule of $O_2$, the equation is the following:

$$2 * \frac{d[H_2O_2]_{GO}}{dt} = \frac{d[O_2]_{CAT}}{dt}$$

Additionally, as a fraction of $H_2O_2$ is being consumed by the working electrode, the amount of $H_2O_2$ that needs to be corrected for from simple diffusion is about 50% of the total amount of $H_2O_2$ produced by glucose oxidase. Therefore, the final balance is:

$$\frac{d[H_2O_2]_{GO}}{dt} = \frac{d[O_2]_{CAT}}{dt}$$

$$\frac{V_{max}^{GO}(Temp, Loading)[G]}{K_M^{GO} + [G]} = \frac{V_{max}^{CAT}(Temp, Loading)[H_2O_2]}{K_M^{CAT} + [H_2O_2]}$$

This suggests that when the reaction rates of glucose oxidase and the oxygen generating enzyme (e.g., catalase) are substantially the same, the effects of temperature variation on the glucose sensor may be canceled out. According to

the invention this can be achieved by varying the loading of the oxygen generating enzyme (e.g., catalase) and/or glucose oxidase on the sensor deposition layers. It is important to note that the glucose oxidase enzymatic layer and the oxygen replenishing layer containing the oxygen generating enzyme (e.g., catalase) must be physically separate and generally follow this order: electrode=>glucose oxidase=> oxygen-generating enzyme.

Since the oxygen-replenishing layer 50 (e.g., the catalase layer) captures the fraction of $H_2O_2$ leaving the CGM sensor and converts it into $O_2$, adding this oxygen-replenishing layer as proposed in FIG. 1B increases the local availability of $O_2$ molecules near the CGM sensor. This increases the extent of linearity of the sensor, in other words increasing the accuracy of the sensor, to a wider range of glucose concentrations (given the concentration of glucose oxidase enzyme loaded onto the sensor is not limiting).

Catalase, owing to its higher turnover rate, will catalyze the decomposition of the fraction of $H_2O_2$ that lingers within the sensor, thereby reducing the oxidation of sensor components and increasing the lifetime of the sensor. Furthermore, by adding the catalase layer, the fraction of $H_2O_2$ that lingers within the sensor is kept to a minimum, if not completely eliminated. This also reduce the undesirable hysteresis of the glucose sensor.

In other embodiments of the oxygen-replenishing layer described herein, catalase can be replaced with any other peroxidases at appropriate concentrations that allow for synergistic functioning with glucose oxidase. Furthermore, the oxygen-replenishing layer may include other enzymes to reduce or eliminate any interfering molecules from diffusing to the electrode. In some embodiments, the oxygen-replenishing layer comprises one or more enzymes selected from the group consisting of peroxidases, transferases, hydrolases, oxidases, kinases, superoxidases, phosphatases, pyrophosphatases, hydroxylases, dioxygenases, dehydrogenases, carboxylases, aminases, catalase, phosphohydrolases, diaminases, reductases, synthases, and caspases, and combinations thereof. In some embodiments, the oxygen-replenishing layer further comprises a glucokinase or a gluconate dehydrogenase to reduce or eliminate the accumulation of gluconolactone. In some embodiments, the oxygen-replenishing layer further comprises further comprises an ascorbate peroxidase or ascorbate oxidase, which can reduce or eliminate interfering molecule ascorbic acid before it reaches to the working electrode. Specifically, adding ascorbate peroxidase to the catalase layer will utilize the outgoing fraction of $H_2O_2$ to reduce interference from ascorbic acid during the operation of the CGM sensor. Interestingly, ascorbate oxidase (redox potential ~+0.2 [V] Vs Ag/AgCl) oxidizes vitamin-C (an interferent) to dehydroxyascorbate (redox potential >+0.9 [V] Vs Ag/AgCl). Thereby eliminating the activity of ascorbic acid or dehydroxyascorbate at the operating potential of the CGM sensor of +0.6 [V].

In some additional embodiments, either or both of the enzymatic layer and the oxygen-replenishing layer may be further modified or doped with comprises one or more oxygen binding proteins or oxygen binding globins, or combinations thereof to increase local concentration of oxygen. In some such embodiments, the oxygen binding protein comprises hemerythrin. In some such embodiments, the oxygen binding globin comprises myoglobin, hemoglobin, or a combination thereof.

## Glucose Sensors Containing Polymeric Mediators

### *Polymeric Mediator in the Glucose Oxidase Enzymatic Layer*

In some embodiments of the glucose monitoring device described herein, the glucose oxidase containing enzymatic layer also comprises one or more polymeric mediators. In some such embodiments, glucose oxidase and the polymeric mediator are present in a hydrogel matrix. In some such embodiments, the hydrogel matrix comprises one or more materials selected from the group consisting of cellulose acetate, chitosan, poly(2-hydroxyethyl methacrylate) (pHEMA), polyethylene glycol diamine, 3,6,9-Trioxaundecanedioic acid, sodium citrate, polyvinyl alcohol and polyethylenimine(PEI), and combinations thereof. In some other embodiments, the hydrogel matrix comprises two or more crosslinked materials described herein. A suitable crosslinking molecule can be poly(ethylene glycol) diglycidyl ether, glycerol diglycidyl ether, glutaraldehyde etc. In further embodiments, the hydrogel matrix may further comprise one or more polymeric materials that render the hydrogel matrix with a negative charge, for example, polymers bears cationic or anionic groups, or salt forms of a polymer containing carboxy groups. For example, the one or more polymeric materials may comprise poly(sodium 4-styrenesulfonate), poly(4-styrenesulfonic acid-co-maleic acid) sodium salt, poly(acrylic acid-co-maleic acid), or poly(vinylsulfonic acid) sodium salt, or combinations thereof.

In some embodiments, the glucose sensor comprises the layers illustrated in FIG. 1C. In this example, glucose oxidase is present with a polymeric mediator, trapped in a hydrogel enzymatic layer 70 as described herein. The hydrogel matrix may be advantageous in terms of lower potential applied for detection of glucose and less or no sensor signals caused by interferent molecules. The polymeric mediator essentially competes with oxygen to oxidize glucose oxidase during the enzymatic oxidation of glucose.

In some examples, the polymeric mediator described herein may have a structure as shown in FIG. 3A, in the enzymatic layer 70 to facilitate electron transfer between the glucose oxidase and the electrode 10 during the sensing reaction. In such cases, the electrode can be of a suitable conductive material, such as carbon, graphite, gold, platinum,

silicon, Pt-Ir alloy, etc. As shown in FIG. 3A, the polymeric mediator has three components: a backbone material, at least one type of linker and at least one type of redox mediator moiety. In some embodiments, the backbone material can be a polymer such as PEI, polyallylamine, cellulose, cellulose acetate, chitosan, poly(acrylic acid), or poly(lactic acid), etc. In some embodiments, the backbone material can be carbon nanofibers, carbon nanotubes or metal nanofibers, etc. The linker (denoted by L in FIG. 3A) may comprise a heteroalkylene chain such as a polyethylene glycol (PEG) chain with repeating ethylene glycol ($-OCH_2CH_2-$) units, or an alkylene chain with repeating methylene units. The number of repeating units can be from 1 to 20, from 2 to 10, from 3 to 8, or from 4 to 7. The redox mediator M can be ferrocene or a derivative thereof, transition metal complexes such as iron-phenanthroline, ruthenium complexes, or organic molecules such as viologens, quinones, etc.

In other examples, the polymeric mediator described herein may have a structure as shown in FIG. 3B. In this example, the polymer backbone may comprise a alkylene chain, or a heteroalkylene chain such as polyethylene glycol chain with the number of repeating ($-OCH_2CH_2-$) units ranging from 1 to 10000, from 2 to 5000, from 5 to 1000, or from 10 to 50. The redox mediator M shown in FIG. 3B can be a mediator molecule such as ferrocene, transition metal complexes such as iron complexes (e.g., iron-phenanthroline), ruthenium complexes, or organic molecules such as viologens, quinones etc. The end group N shown in FIG. 3B can be a functional group that can either be a cation or anion or a neutral molecule that improves or increase the water solubility of the polymer molecule. Some of the cation and anion groups can be $-SO_3^-$, $-PO_3^-$, $-NH_3^+$, $-N(CH_3)_3^+$, etc. Some of the neutral end groups that can render the water solubility can be glucose, sucrose, lactose etc. In some cases, the end group N can be a reactive linker. In other cases, the end group N can be the same type or a different type of a redox mediator molecule similar to the end group M.

In some embodiments, the enzymatic layer comprising the glucose oxidase and the polymeric mediator may further comprise a second enzyme. In some embodiments, the second enzyme is a peroxidase, for example, horseradish peroxidase. In another embodiment, the second enzyme is catalase.

### *Glucose Oxidase Containing Enzymatic Layer*

In any embodiments of the glucose monitoring devices described herein, glucose oxidase may be captured onto the glucose sensor by various methods. Typically, this step is performed in aqueous solvent because of the enzymatic nature of glucose oxidase.

### A. Physical adsorption of the enzyme and crosslinking by glutaraldehyde

As an example, a multi-layer deposition method may be used by repeated physical adsorption of the GOx in the presence of bovine serum albumin (BSA). The efficiency of deposition glucose oxidase enzyme on the sensor can be improved by co-capturing it with bovine serum albumin (BSA). Additionally, BSA provides structural stability as the outer surface of BSA is decorated by lysine residues that act as sites for cross-linking other primary amines when exposed to glutaraldehyde.

In one example, on a wire-based electrode, glucose oxidase was deposited by alternate dipping in glucose oxidase and bovine serum albumin solution in phosphate buffered saline (PBS) at pH 6.5 and 10% glutaraldehyde solution in PBS at pH 6.5. The dipping procedure was performed using a commercial dip-coating instrument with precise control of dip and withdrawal speed, timing of the dip, waiting time between dips and stirring of the dipping solutions. These parameters are important to ensure repeatable and consistent deposition of the enzyme on the electrode.

It has been empirically observed that BSA facilitates consistency and loading density of glucose oxidase on the sensors during dipping. BSA has 30 or more lysine residues with primary amine groups uniformly distributed on its surface, compared to about 7 lysine residues on glucose oxidase enzyme. The high availability of lysine on BSA's surface enables for robust crosslinking/capturing glucose oxidase by glutaraldehyde. Additionally, glutaraldehyde might also allow crosslinking BSA and glucose oxidase enzyme to the underlying PoPD layer that might have unreacted primary amine groups in its polymer network.

In another example, on a disk based electrode, glucose oxidase and BSA was mixed with glutaraldehyde and deposited by drop-casting. The electrodes were dried under vacuum for 1 hour at room temperature. Immediately after, the electrodes were washed in PBS at pH 7.4 for 30 minutes with stirring to remove uncrosslinked/loosely bound glucose oxidase and BSA.

### B. Layer-by-Layer deposition based on electrostatic interaction

As another example, a layer-by-layer deposition of glucose oxidase based on electrostatic interaction of alternating negatively and positively charged layers. The total charge on glucose oxidase molecule is negative (pI 4.3) at pH 7.4. Using this property, glucose oxidase can be electrostatically captured on the surface of the electrode that has previously prepared to have positively charged molecules.

C. Co-electropolymerization with glutaraldehyde fixing

Another way to capture glucose oxidase on the sensor is co-electropolymerization of glucose oxidase and ortho-phenylenediamine (oPD) followed by glutaraldehyde fixing. In this procedure, an electrochemical cell containing a solution of oPD monomers and glucose oxidase enzyme (1-5 mg/ml) is subjected to a +0.7 [V] vs Ag/AgC1 at the working platinum electrode for about 15 min. Applying this positive potential starts the electro-polymerization of oPD monomers to PoPD near the electrode surface. Because of the presence of glucose oxidase molecules (ca 8 nm in size) in the solution, they get "captured" during this process onto the electrode surface within the PoPD "matrix". With this procedure about 3.5 Units of GOx per $cm^2$ can be captured on the electrode, which is capable to generate currents of ca. 5 $\mu A/cm^2$ mM and high $K_M$ = 16 mM). Subsequently, the glucose oxidase molecules are crosslinked to each other (maybe also to PoPD) by immersing the sensor into 2.5% glutaraldehyde (GA) for 30 mins. GA is potent crosslinker that adds covalent bonds between primary amine groups (like sidechains of lysine and arginine residues and exposed/un-polymerized amine groups in the PoPD polymer). This step "fixes" the glucose oxidase molecules to the sensors by covalent bonds. However, non-selective capturing of glucose oxidase during the electro-polymerization of oPD molecules is possibly still highly variable. Other phenylene diamine such as mPD and pPD may also be used as replacement to oPD or in combination with oPD in the co-electropolymerization. In addition, other electro-polymerizable monomers like pyrrole or aniline may also be used alone or in combination with the phenyl diamine described herein for increasing the robustness of the co-electropolymerization,

oPD to PoPD polymerization reaction is highly dependent on dissolved oxygen content, pH of the solution, doping of the solution ($H_2SO_4$ vs HCl vs $HNO_3$), electrolyte/salt concentration and temperature of polymerization. These conditions determine the uniformity of PoPD polymer layer. The concentrations of these variables are controlled by buffer preparation in deionized water. Furthermore, other monomers may also be used in the electropolymerization, including but not limited to o-phenylene diamine, pyrrole, aniline, aniline, sulfonated aniline, sulfonated thiophenes, flavin mononucleotide, substituted anilines, substituted pyrroles, substituted thiophenes, acetylenes, polyethylene dioxythiophenes, ethylenedioxypyrroles, phenylene vinylenes, carbazoles, substituted carbazoles, indoles, carboxy-functionalized aqueously dispersed carbon nanotubes, flavin mononucleotide coated single wall carbon nanotubes, aqueous dispersed nanoparticles with aniline functionalities, and combinations thereof.

An important factor for the reliable performance of the glucose sensor is the local concentration of glucose and oxygen near the GOx enzymatic layer. The oxygen levels in the interstitial fluid (the location of implanted sensor) can range from 1-4 kPa (0.4 mM to 1.6 mM) under normoxia conditions or 0.1-1 kPa (0.04 mM to 0.4 mM) under moderate hypoxia conditions. However, the concentration of glucose in the interstitial fluid ranges from 40-400 [mg/dl] (2.2 mM to 22.2 mM). Given that glucose oxidase consumes equal molar amounts of glucose and $O_2$ to generate equal molar amount of $H_2O_2$, the current response from oxidizing $H_2O_2$ at the working electrode is proportional to the glucose concentration only if the $O_2$ concentration is well above the glucose concentration in the local region next to glucose oxidase enzyme in the sensor. Thus, to ensure a linear glucose-dependent current response of the sensor, the glucose concentration needs to be limited to a maximum of 0.4 mM (lower limit of $O_2$ under normoxia conditions) at or near the glucose oxidase enzyme layer of the sensor. This represents a 98.2% reduction compared to endogenous glucose levels. As such, the first permeability-selective layer described herein serves to selectively limit the diffusion of glucose while being fully permeable to $O_2$. In addition, the oxygen generated by the enzyme in the oxygen-replenishing layer serves to increase the local concentration of oxygen in the GOx enzymatic layer. The synergistic effects of the two layers optimizes the performance of the glucose monitoring device described herein.

*First Permeability-Selective Layer*

The first permeability-selective layer, such as layer 40 illustrated in FIG. 1B and FIG. 1C, is designed to equalize the concentrations of glucose and oxygen molecules at the GOx enzymatic layer. Equal molar concentration of oxygen and glucose required for glucose oxidase catalyzed reaction of the substrate glucose. However, the concentration of oxygen dissolved in the interstitial fluid (the location of implanted sensor), can be as low as $1/100^{th}$ the concentration of glucose. This consequently leads to a non-linear response of glucose sensor to increase in glucose concentration, eventually reaching saturation. Keeping the sensor response in the linear region is critical for increased accuracy of the CGM sensor across the physiological glucose concentration range. Therefore, a permeability-selective outer layer is employed to selectively block glucose molecule from diffusing towards the electrode in order to keep the ratio of oxygen to glucose molecules at the GOx layer to a value of at least 1 or higher for all glucose concentrations.

The first permeability-selective layer (i.e., the outer permeability selective layer) physically and chemically blocks the accessibility of glucose while allowing unhindered diffusion of oxygen. The control of the pore-size and chemical composition of this layer determines the efficiency and specificity of this layer's primary functionality.

In some embodiments, the first permeability-selective layer comprises one or more polymers selected from the group consisting of a polyacetal, a polyolefin, a polyacrylic, a polycarbonate, a polystyrene, a polyester, a polyamide, polyamideimides, a polyarylate, a polyarylsulfone, a polyethersulfone, a polyphenylene sulfide, a polyvinyl chloride, a

polyethylene oxide, a polysulfone, a polyimide, a polyetherimide, a polytetrafluoroethylene, a polyetherketone, a polyether etherketone, a polyether ketone ketone, a polybenzoxazole, a polyphthalide, a polyacetal, a polyanhydride, a polyvinyl ether, a polyvinyl thioether, a polyvinyl alcohol, a polyvinyl ketone, a polyvinyl halide, a polyvinyl nitrile, a polyvinyl ester, a polysulfonate, a polysulfide, a poly(allyl amine), a polythioester, a polysulfone, a polysulfonamide, a polyurea, a polyphosphazene, a polysilazane, a polyvinylchloride, a polyvinyl acetate, a humic acid, a cellulose acetate, a polythiophene, a polyphenylene diamine, a polypyrrole, a polynaphthalene a polyurethane, an ethylene propylene diene rubber, a polytetrafluoroethylene, a fluorinated ethylene propylene, a sulfonated tetrafluoroethylene based fluoropolymer-copolymer (e.g., Nafion™), a perfluoroalkoxyethylene, a polychlorotrifluoroethylene, a polyvinylidene fluoride, and a polysiloxane, and combinations thereof. In some embodiments, the first permeability layer comprises or is a layer of poly(orthophenylenediamine) (PoPD), poly(meta-phenylenediamine) (PmPD), or poly(para-phenylenediamine) (PpPD), or combinations thereof. In some such embodiments, one or more layers can be formed by polymerization of one or more of phenylene diamine (such as oPD, mPD, or pPD), or other polymerizable monomers such as pyrrole, or aniline, or combinations thereof. The polymerization method can either be performed by electrochemical means at a preferred electrode material or by chemical means at an electrode material. In some embodiments a pre-polymerized material can be deposited on the surface by a suitable deposition method. In one embodiment, the first permeability-selective layer comprises Nafion™,

In some embodiments of the multi-enzyme temperature independent glucose monitoring device described herein, the first permeability-selective layer is disposed between the enzymatic layer and the oxygen-replenishing layer. In some further embodiments, the first permeability-selective layer is in direct contact with one or both of the enzymatic layer and the oxygen-replenishing layer. In some other embodiments, the first permeability-selective layer is disposed between the oxygen-replenishing layer and the outer protective layer. In some further embodiments, the first permeability-selective layer is in direct contact with one or both of oxygen-replenishing layer and the outer protective layer.

In some other embodiments of the glucose monitoring device described herein, the first permeability-selective layer is disposed between the enzymatic layer containing glucose oxidase and a polymeric mediator and the outer protective layer. In some further embodiments, the first permeability-selective layer is in direct contact with one or both of the enzymatic layer and the outer protective layer.

*Second Permeability-Selective Layer*

In some embodiments, any aspect of the glucose monitoring devices described herein further comprises a second permeability-selective layer (such as the inner permeability-selective layer 20 illustrated in FIG. 1B) for blocking the contact of one or more redox active species with the working electrode and/or the reference electrode. In some such embodiments, the second permeability-selective layer is disposed between the working electrode 10 and the glucose oxidase containing enzymatic layer 30 as shown in FIG. 1A and FIG. 1B, or glucose oxidase and polymeric mediator containing enzymatic layer 70 as shown in FIG. 1C. In some further embodiments, the second permeability-selective layer is in direct contact with one or both of the working electrode and the enzymatic layer. The function of the first permeability-selective layer (inner permeability-selective layer) is to prevent interference from endogenous and exogenous redox active species at the operating potential of the electrode (e.g., +0.6 V). In some embodiments, the one or more redox active species comprises endogenous or exogenous compounds or metabolites present in a mammal's bodily fluid, tissue fluid, or serum. In some further embodiments, the one or more redox active species comprises ascorbic acid, uric acid, or acetaminophen, or combinations thereof. The filtering of unwanted redox active species can be achieved by selectively limiting interfering molecules by their chemical properties and/or by their size (by controlling the pore size of the second permeability-selective layer). The first permeability-selective layer does not block or prevent $H_2O_2$ to reach the electrode.

In some embodiments, the second permeability-selective layer comprises electropolymerized poly(orthophenylenediamine) (PoPD), electropolymerized poly(meta-phenylenediamine) (PmPD), electropolymerized poly(para-phenylenediamine) (PpPD), cellulose acetate, or an ionic polymer (e.g., Nafion™), or combinations thereof. In some embodiments, the second permeability-selective layer has a thickness from about 1 nm to about 10 $\mu$m, from about 2 nm to about 1 $\mu$m, or from about 5 nm to about 500 nm. In some further embodiments, the second permeability-selective layer has a thickness of about 10 nm to about 300 nm. In one embodiment, the second permeability-selective layer comprises electropolymerized PoPD having a thickness ranging from about 50 nm to about 500 nm, from about 100 nm to about 300 nm, or from about 150 nm to about 230 nm. In another embodiment, the second permeability-selective layer comprises electropolymerized PmPD having a thickness ranging from about 10 nm to about 100 nm, from about 20 nm to about 50 nm, from about 25 nm to about 40 nm, or about 30 nm. In yet another embodiment, the second permeability-selective layer comprises electropolymerized PpPD having a thickness ranging from about 200 nm to about 5 $\mu$m, from about 500 nm to about 2 $\mu$m, or about 1 $\mu$m.

PoPD/PmPD/PpPD is formed on the surface of the Pt/Ir electrode from an aqueous solution of ortho-phenylenediamine (oPD), meta-phenylenediamine (mPD) or para-phenylenediamine (pPD) monomers, respectively by the process of electrochemical polymerization when a positive potential is applied to the working electrode. A thin film is

formed (for example, from about 10 to about 300 nm in thickness, depending on the monomers used) that serves as an efficient barrier to undesired electrochemically active interferences such as ascorbate and acetaminophen. PoPD, PmPD and PpPD have varying degree of perm-selectivity towards hydrogen peroxide and other interfering species (such as ascorbate, uric acid, acetaminophen, etc.) It has been surprisingly discovered that PmPD based interference blocking layer (i.e., second permeability-selective layer) provides the highest permeation selectivity with nearly about 100% blocking of ascorbic acid and acetaminophen, while allowing at least about 60% of hydrogen peroxide to diffuse through the PmPD layer to the electrode surface (with respect to the bare Pt electrodes). A typical concentration of oPD is 100 mM in phosphate buffered saline (PBS) at pH 7.4.

One preferred method of electropolymerization of oPD/mPD/pPD is amperometry because cyclic voltammetry-based deposition results in polymers with a higher permeability towards interfering redox active molecules. The second permeability-selective layer may be deposited directly on the surface of the electrode. In one embodiment, the thickness of second permeability-selective layer comprising or made of PoPD is about 150m to about 230 nm. In one embodiment, the thickness of second permeability-selective layer comprising or made of PmPD is about 30 nm. In another embodiment, the thickness of second permeability-selective layer comprising or made of PpPD is about 1 $\mu$m. PmPD performs the best with selectivity of $H_2O_2$ with respect to the other interfering species. With near complete blocking of ascorbic acid and acetaminophen while still maintaining 60% or more permeability to $H_2O_2$ (with respect to the bare Pt electrode) and the thinnest deposition profile of all three polymeric membranes (i.e., PoPD, PmPD, and PpPD).

In addition to the polymers formed from phenylenediamine monomers described herein, diamino-naphthalene analogs and amino naphthol analogs may also be used in preparing the second permeability-selective layer. In one example, polymer prepared from 2,3-diamino-napthalene (p-2,3-DAN) is observed to have excellent blocking (near zero) of ascorbic acid, acetaminophen and urate molecules while maintaining some selectivity to $H_2O_2$. In some embodiments, p-2,3-DAN has an average thickness of about 100 nm to about 300 nm, or about 120 nm to about 200 nm, or about 150 nm. In some further embodiments, p-2,3-DAN has a permeability to $H_2O_2$ at about 30% (with respect to the bare Pt electrode). As another example, polymerized 5-amino-naphthol (p-5-AIN) has an average thickness of about 40 nm to about 150 nm, or about 60 nm to about 100 nm, or about 70 nm, and also has complete or nearly complete blocking of interfering molecules and 20% $H_2O_2$ permeability (with respect to the bare Pt electrode). Other non-limiting examples of the monomers that may be used in the electrochemical polymerization to form a second permeability-selective layer include 1,5-diamino-napthalene (1,5-DAN), 1,8-diamino-napthalene (1,8-DAN), polypyrrole (PPy), and polyaniline (PANI). These may be used either alone, or in combinations with the other monomers described herein to form the second permeability-selective layer.

Non-uniform and inconsistent application of voltage across all electrodes, temperature control and availability (diffusion characteristics) of oPD monomers at the electrode during its polymerization is the main source of variability. In some embodiments, purging the dissolved oxygen from monomers dissolved in PBS is important in achieving reproducibility.

In another embodiment, alternating layers of 6% cellulose acetate (CA) and 5% Nafion™ are dip-coated to ensure uniform, non-undulating molecular layers of these two materials. The percentages of each material (6% cellulose acetate and 5% Nafion™) determines the mechanical pore size along with the functional group density in each layer. A single deposition of double-layer of CA/Nafion™ eliminated majority of interfering molecules, however, this can be extended to multiple alternating double-layers of CA/Nafion™ for further elimination of interference. Functionally, the cellulose acetate layer blocks interfering species mechanically by having a certain pore size dictated by the concentration of cellulose acetate. The CA layer has been reported to be able to effectively blocks acetaminophen. Nafion™ is a sulfonated tetrafluoroethylene based fluoropolymer-copolymer that is negatively charged and can effectively blocks uric acid and ascorbic acid (both of which are present in the anionic or salt form such as urate and ascorbate under physiological conditions).

*Outer Protective Layer*

The addition of the outer protective layer 60 in the glucose sensor illustrated in FIG. 1B and FIG. 1C is primarily for the purpose of improving the mechanical stability of the sensor and decreasing undesired interaction between the sensor the biological medium, such as protein adhesion. In some embodiments, the outer protective layer is biocompatible.

All implanted devices perturb the physiological environment and initiate a biological response. This can lead to lowered sensitivity of the sensor *in vivo* compared to its *in vitro* values. Acute inflammatory response starts immediately after the sensor is implanted. During which, fluid carrying plasma proteins and inflammatory cells migrate to the site of implant. Proteins are adsorbed initially and then phagocytic cells (neutrophils, monocytes, and macrophages) surround the biosensor and attempt to destroy it. Because a biosensor is large compared to the phagocytic cells, they unsuccessfully attempt to ingest the sensor. They also release reactive oxygen species [ROS ($H_2O_2$, $O_2^-$ , NO, $OH^-$)] and enzymes intended to degrade the implant. The exact timing, action, and intensity of the process are dependent on the nature of the foreign body, which relates to size, shape, and physical and chemical properties. The acute response lasts about 3 days

after which a chronic inflammatory response may set in or a modified version of the healing process begins. Ultimately a fibrotic capsule is formed, which is the hallmark of the foreign body response.

In the case of glucose sensors, there is a possibility that the inflammatory response affects the concentration of glucose in the immediate vicinity of the sensor. This may be due to changes in the diffusion characteristics of the tissue because of the inflammatory response, or due to the formation of a fibrotic capsule surrounding the sensor implant. In the literature, it has also been suggested that insufficient vascularization surrounding the implanted sensor decreases appropriate glucose concentration at the sensor implant site, and that this is alleviated after a few days when angiogenesis has produced new capillaries. Improved neovascularization by incorporating an angiogenesis factor such as vascular endothelial growth factor (VEGF) or adding a specially structured polytetrafluoroethylene (PTFE) membrane on the sensor surface has been reported. The straightforward method to elimination of inflammatory response is using anti-inflammatory drugs such as dexamethasone, nitric oxide (NO) within the sensor itself. For example, it has been reported that PVA composite with microsphere filled with dexamethasone for slow and prolonged release after implantation to prevent inflammation.

In some embodiments, the outer protective layer comprises a polymer, a hydrogel, or a combination thereof. In some such embodiments, the outer protective layer comprises polyvinyl alcohol (PVA), Nafion™, or a combination thereof. In one embodiment, the outer protective layer comprises crosslinked PVA. These materials are intended to inhibit protein adhesion and therefore reduces possible inflammatory response. In some further embodiments, the outer protective layer further comprises an anti-inflammatory drug, an angiogenesis factor, or a combination thereof.

## *Configurations of the Temperature Independent Glucose Sensor Layers*

In some embodiments of the temperature independent glucose monitoring device described herein, the second permeability-selective layer is disposed between the working electrode and the enzymatic layer, the enzymatic layer is disposed between the second permeability-selective layer and the first permeability-selective layer, the first permeability-selective layer is disposed between the enzymatic layer and the oxygen-replenishing layer, and the oxygen-replenishing layer is disposed between the first permeability-selective layer and the outer protective layer. In some further embodiments, the second permeability-selective layer is disposed between and in direct contact with either or both the working electrode and the enzymatic layer, the enzymatic layer is disposed between and in direct contact with either or both the second permeability-selective layer and the first permeability-selective layer, the first permeability-selective layer is disposed between and in direct contact with either or both the enzymatic layer and the oxygen-replenishing layer, and the oxygen-replenishing layer is disposed between and in direct contact with either or both the first permeability-selective layer and the outer protective layer.

In some other embodiments of the temperature independent glucose monitoring device described herein, the second permeability-selective layer is disposed between the working electrode and the enzymatic layer, the enzymatic layer is disposed between the second permeability-selective layer and the oxygen-replenishing layer, the oxygen-replenishing layer is disposed between the enzymatic layer and the first permeability-selective layer, and the first permeability-selective layer is disposed between the oxygen-replenishing layer and the outer protective layer. In some further embodiments, the second permeability-selective layer is disposed between and in direct contact with either or both the working electrode and the enzymatic layer, the enzymatic layer is disposed between and in direct contact with either or both the second permeability-selective layer and the oxygen-replenishing layer, the oxygen-replenishing layer is disposed between and in direct contact with either or both the enzymatic layer and the first permeability-selective layer, and the first permeability-selective layer is disposed between and in direct contact with either or both the oxygen-replenishing layer and the outer protective layer.

In some other embodiments, the second permeability-selective layer (inner selective layer) is not present. The enzymatic layer is disposed between the working electrode and the first permeability-selective layer, the first permeability-selective layer is disposed between the enzymatic layer and the oxygen-replenishing layer, and the oxygen-replenishing layer is disposed between the first permeability-selective layer and the outer protective layer. In some further embodiments, the enzymatic layer is disposed between and in direct contact with either or both the working electrode and the first permeability-selective layer, the first permeability-selective layer is disposed between and in direct contact with either or both the enzymatic layer and the oxygen-replenishing layer, and the oxygen-replenishing layer is disposed between and in direct contact with either or both the first permeability-selective layer and the outer protective layer.

In some other embodiments, the second permeability-selective layer (inner selective layer) is not present. The enzymatic layer is disposed between the working electrode and the oxygen-replenishing layer, the oxygen-replenishing layer is disposed between the enzymatic layer and the first permeability-selective layer, and the first permeability-selective layer is disposed between the oxygen-replenishing layer and the outer protective layer. In some further embodiments, the enzymatic layer is disposed between and in direct contact with either or both of the working electrode and the oxygen-replenishing layer, the oxygen-replenishing layer is disposed between and in direct contact with either or both the enzymatic layer and the first permeability-selective layer, and the first permeability-selective layer is disposed between

and in direct contact with either or both the oxygen-replenishing layer and the outer protective layer.

FIG. 2 is an exemplary schematic illustration of a temperature independent glucose monitoring device in a specific layer configuration described herein. It comprises a Pt working electrode; a second permeability-selective layer comprising alternating layers of cellulose acetate and Nafion™ in direct contact with the Pt electrode; a GOx enzymatic layer resulted from co-electropolymerization of glucose oxidase and ortho-phenylenediamine (oPD) where the GOx layer is sandwiched between the second permeability-selective layer and a first permeability-selective layer comprising Nafion™; an oxygen-replenishing layer comprising catalase; and an outer protective layer comprising PVA or Nafion™.

*Configurations of the Polymeric Mediator Containing Glucose Sensor Layers*

In some embodiments, the first permeability-selective layer is disposed between the enzymatic layer (containing the glucose oxidase and the polymeric mediator) and the outer protective layer. In some further embodiments, the first permeability-selective layer is in direct contact with one or both of the enzymatic layer and the outer protective layer. In some embodiments, the device further comprises a second permeability-selective layer for blocking the contact of one or more redox active species with the working electrode and/or the reference electrode. In some such embodiments, the second permeability-selective layer is disposed between the working electrode and the enzymatic layer. In some further embodiments, the second permeability-selective layer is in direct contact with one or both of the working electrode and the enzymatic layer. In some further embodiments, the second permeability-selective layer is disposed between the working electrode and the enzymatic layer, the enzymatic layer is disposed between the second permeability-selective layer and the first permeability-selective layer, the first permeability-selective layer is disposed between the enzymatic layer and the outer protective layer.

In any embodiments of the various layers of the glucose monitoring device described herein, each layer may also comprises multiple sublayers, depending on the manufacturing methods used. For example, the second permeability-selective layer described herein may include alternating layers of cellulose acetate (CA) and Nafion™ formed by dip coating.

In any embodiments described herein, any one or more of the first permeability-selective layer, the enzymatic layer, the oxygen-replenishing layer, the second permeability-selective layer, and the outer protective layer may further comprises one or more enzymes for reducing or eliminating the interference of one or more interfering molecules with the working electrode. In some such embodiments, the one or more interfering molecules comprise ascorbic acid, uric acid, or acetaminophen, hydroxyurea, cholesterol, creatinine, dopamine, ethylenediaminetetraacedic acid (EDTA), gentisic acid, heparin, or salicylic acid, or combinations thereof.

*Electrodes*

Any embodiments of the glucose monitoring device described herein comprises at least two electrodes - the working electrode and the reference electrode. In some embodiments, the working electrode and/or the reference electrode comprises one or more conductive materials, such as metals. In some further embodiments, the working electrode and/or the reference electrode comprises platinum (Pt), gold (Au), silver (Ag), rhodium (Rh), iridium (Ir), or combinations thereof. In one embodiment, the working electrode comprises Pt. In another embodiment, the working electrode comprises both Pt and Ir. In one embodiment, the reference electrode comprises silver and silver chloride. In other embodiments, the electrodes may contain non-metal materials such as graphite, glassy carbon, carbon fiber, silicon (such as p-doped or n-doped silicon), etc. In some embodiments, the device further comprises a counter electrode. The counter electrode may comprises one or more metals described herein. In one embodiment, the counter electrode comprises Au.

The surface characteristics of Pt or Pt/Ir working electrodes are important for all subsequent steps of fabrication of the CGM sensor. Any deposits of organic material, chemical impurities and oxidized metal can lead to irregular electrical conductivity along the surface due to different surface adsorption characteristics towards the analyte (for electrochemical deposition, electro-polymerization, electrochemical area determinations, etc.); irregular physical adsorption of *inner selective* layers (PoPD, cellulose acetate, Nafion™, polyphenol etc.); or irregularities in distribution of the hydroxyl groups on the Pt wire surface, which is critical for uniform oxidation rates of $H_2O_2$. There are various protocols of cleaning the surface of Pt/Ir, including but not limited to: a) mechanical agitation in 100% acetone and deionized water using titanium-tip sonication; b) soaking in concentrated nitric acid to dissolve residual organic matter and to etch the platinum surface slightly; and c) electrochemical conditioning / activation of the surface by performing multiple cycles of cyclic voltammetry between -0.2 [V] to 1.145 [V] in 1 M sulfuric acid ($H_2SO_4$).

Therefore, the electrochemical oxidation reactions of most redox species are preferred at potentials close to those of the platinum oxide (Pt(O)) surface formation for greatest response.

Electrochemical species undergo electro catalytic oxidation at the Pt surface through the reaction with oxygen, which may come from bulk water or from surface oxides on the electrode formed by anodic activation of the Pt surface.

Having the oxide layer on the Pt surface accelerates the electrochemical charge transfer reactions because of readily available platinum oxide. In the first step of this cyclic mechanism, $H_2O_2$ reacts with the surface of Pt to form Pt(O), releasing one molecule of $H_2O$. In the second step, a second molecule of $H_2O_2$ reduces Pt(O) to metallic Pt, releasing a second molecule of second $H_2O$ and $O_2$. The first reaction is a rate-limiting step in this two-part reaction. Incorporating Pt(O) at the surface (activation) exhibit a faster rate of $H_2O_2$ decomposition because the rate limiting step of the reaction is skipped in the first cycle.

There are many potential parallel/competing Pt based $H_2O_2$ electrochemical oxidation reaction mechanisms. The anodic activation of Pt can be achieved by application of cyclic voltammetry, where the electrode is anodized by scanning the potential in the anodic region and/or holding the potential for some time at the anodic limit. In one example, during Pt cleaning procedure with 1 M $H_2SO_4$, the cyclic voltammetry scans were stopped at the final high anodic potential of 1.145 V vs Ag/AgCl. At this potential a uniform Pt(O) layer and an activated Pt/Ir surface is formed.

To detect $H_2O_2$, GOx enzymatic layer will be deposited on the platinum electrode, along with other layers for normalizing the glucose signal, which will act as the working electrode (WE). A three-electrode configuration includes two other electrodes; a solid-state silver/silver chloride (Ag/AgCl) as a reference electrode (RE) against which the potential of the working electrode is maintained at a constant value; and a counter electrode (CE) made of any stable/noble metal (Au, Pt, stainless steel or others) which acts as a conduit to pass the current between the working electrode and itself. When the working electrode surface is held at the operating potential of the CGM (+0.6V) with respect to Ag/AgCl, $H_2O_2$ oxidizes to $O_2$ and releases two electrons per molecule of $H_2O_2$.

$$2AgCl + 2e^- \rightarrow 2Ag + 2Cl^-$$

However, at this potential other endogenous redox active species such as ascorbic acid, uric acid as well as exogenous redox active species such as acetaminophen also undergo reduction/oxidation at the electrode surface. This leads to an increase or decrease in the amperometric signal, and this is one of the main limitations of a first generation sensor. As described herein, the incorporation of a second permeability-layer and/or an oxygen-replenishing layer can substantially reduce or eliminate the signal caused by the interfering by blocking these interfering species from reaching the electrode surface. As described herein, CGM sensor interference can be reduced/eliminated by adding a combination of enzymes that specifically catalyze the decomposition of the following interfering molecules in the outer layers of the sensor before they reach the electrode: ascorbic acid, uric acid, or acetaminophen, hydroxyurea, cholesterol, creatinine, dopamine, ethylenediaminetetraacedic acid (EDTA), gentisic acid, heparin, or salicylic acid, or combinations thereof. The enzymes specific for each of the interfering molecules may be add to one or more of the second-permeability selective layer (inner selective layer), the GOx enzymatic layer, the first-permeability selective layer (outer perm-selective layer), or the outer protective layer.

*Conditions, Metrics and Variables for Sensor*

1. Fabrication and Storage

In some of the working examples described herein, experiments were conducted over two physical/geometrical construction of sensors. The first type of construction is platinum wire based sensors with cylindrical geometry (~0.85 [$mm^2$] area). The second type of construction is platinum disk based sensors with flat circular geometry (2 [$mm^2$] area). The conditions for deposition of each layer is dependent on the geometry. For example, disk electrodes are not compatible with dip-coating method for depositing glucose oxidase, the oxygen replenishing layer, or the first/second permeability-selective layer. For each type of construction, drop-casting with controlled volume/mass of each layer was adopted appropriately.

All experimental results described herein were collected over six physically distinct electrodes that were fabricated in a batch wise manner. All electrodes, at various stages of layer deposition were stored at room temperature (about 22-23°C), enclosed within glass vials at the end of each day or for long-term storage.

2. Measurement Conditions

All results described herein were based on measurements performed in a 150 ml, water-jacketed electrochemical cell, that was maintained at 23 ± 0.1°C. The results described herein were analyzed in the diffusion regime, in static, non-stirred solutions within the electrochemical cell. In particular, the molecular tracers for layer-by-layer characterization were added to the cell, stirred to homogenize the solution and wait for all mixing convection to settle before calculating statistics on current response to various tracer molecules. The area of each electrode was measured geometrically using a calibrated microscope to determine the assumed electrochemically active surface. For the wire based electrodes, the diameter of the wire was 0.125 [mm] (as manufactured) with varying lengths, with an intended length

of 2 [mm], but the actual length was precisely measured using a calibrated microscope. For disk electrodes, the geometrical surface area was assumed to manufacturer's specifications. The permeability assessment of molecular tracers (disk electrode) including the following: hydrogen peroxide (37% wrt. bare electrode); acetaminophen (5% wrt. bare electrode); ascorbic acid (0.7% wrt. bare electrode).

Some additional aspect of the present disclosure relates to a method of implanting a glucose monitoring device to a subject in need thereof, comprising: contacting a glucose monitoring device described herein with an aqueous medium; and implanting the glucose monitor into a tissue of the subject. In some embodiments, the glucose monitoring device described herein is exposed to a small volume of aqueous medium (e.g., saline) that may lead to swelling of the glucose oxidase enzyme-containing layer, before it is implanted into the tissue of interest. This may be advantageous in rapid establishment of electrical contact between electrodes and also establishing a liquid contact between the sensor layer and the tissue region. This method is expected to reduce the time required to operate the sensor and obtain sensor data.

Additional non-limiting embodiments of the glucose monitoring devices and systems are described in details below.

## Control System Components

FIGs. 4A-4C illustrate an example closed loop environment 100 in which the administration of an insulin formulation may occur. For example, a closed loop environment 100 may include a user 101, one or more sensor devices 110, one or more user devices 102, a network 104, and a backend system 106, wherein at least one sensor device is described herein.

A user 101 may interact with the one or more sensor devices 110 directly or through one or more user devices 102. The one or more user devices 102 may include a smart device, such as a smart watch, smart phone, tablet, computer, the like or a combination thereof. It should be noted that a user's mobile device, such as a smart phone, may or may not be considered a permanent communication line. In some examples, a user's mobile device, such as a phone, may not be required by the embedded closed loop system to keep the user in tight glycemic control.

In some examples, the one or more user devices 102 may communicate with a sensor device 110 and/or a backend system 106 through a network 104. For example, a user device 102 may receive data from a user 101, such as a time and composition of a user intake of food. The user device 102 may communicate the data, through the network, to the backend system 106. The backend system 106 may then transmit information based on the received data to the user device 102. In some examples, a user device 102 may directly communicate with a sensor device 110 through wires or wirelessly. In some examples, a wireless mode of communication can include, but is not limited to, WiFi, NFC or Bluetooth connection.

In some examples, one or more components of a closed loop system may include at least one sensor device 110 comprising a glucose sensor or glucose monitor device as described herein. A sensor device 110 may be configured to upload and/or receive data through the user device 102 or the network 104. As illustrated in FIG. 4B, in some examples, one or more hardware components may include two sensor devices 110A, 110B, such as a pair of continuous glucose monitors. In some examples, sensor devices 110 may include a primary sensor device 110A and a secondary sensor device 110B. Advantageously, this may allow for redundancy and staggered active use of glucose sensors so as to allow for at least one glucose sensor 110 to be active and calibrated at any given time during use of the redundant staggered system. In some examples, one or more sensor devices 110A, 110B may include a single sensor device and an insulin pump. In some examples, one or more sensor devices 110A, 110B may include a combined glucose sensor and insulin dosage system 111, such as illustrated in FIG. 4C and as described in U.S. Publication No. 2021/0236729.

## Example Disease Management System

FIG. 5 shows a block diagram of an example disease management system (e.g., prediabetes, Type 1 diabetes, or Type 2 diabetes) 1101, which includes the insulin formulation described herein. In some examples, the disease management system 1101 may be part of a disease management environment, such as described above. A disease management system 1101 may be configured to measure one or more physiological parameters of a patient (such as pulse, skin temperature, or other values), measure one or more analytes present in the blood of a patient (such as glucose, lipids, or other analyte) and administer medication (such as insulin, glucagon, or other medication). In some examples, a disease management system 1101 may be configured to communicate with one or more hardware processors that may be external to the disease management system 1101, such as a cloud based processor or user device. A disease management system 1101 may include an NFC tag to support authentication and pairing with a user device (for example, smart phone or smart watch), Bluetooth communication with additional disease management systems or devices, and Bluetooth communication with a paired user device running an associated control application. To support ease of use and safe interaction with the patient, the system may incorporate user input through a tap-detecting accelerometer and provide feedback via an audio speaker, haptic vibration, and/or optical indicators. The system may operate on battery power and

support both shelf-life and reliable operation once applied to the patient. Battery life may be managed through control of several planned levels of sleep and power consumption. To support this reliability, a controller can monitor several system-health parameters, and monitor temperatures of the included medication, and ambient temperature for the life of the device.

As illustrated in FIG. 5, a controller 1138 of the disease management system 1101 may be configured to communicate and control one or more components of the disease management system 1101. The controller 1138 may include one or more hardware processors, such as a printed circuit board (PCB) or the like. The controller 1138 may be configured to communicate with peripheral devices or components to support the accurate measurement of physiological parameters and blood analytes, such as patient pulse, temperature, and blood glucose, using detector electronics. The controller 1138 may subsequently calculate dose or receive a calculated dose value and administer medication, such as a insulin formulation described herein, by actuation of an actuated pump. The controller 1138 may record device activity and transfer the recorded data to non-volatile secure memory space. At the end of the life of a device or system, the controller can be configured to lock operation, and create a data recovery module to permit authenticated access to the recorded data if needed.

A disease management system 1101 may include an analyte sensor 1120, such as a glucose sensor described herein. The analyte sensor 1120 may be configured to detect analytes in the patient's blood. For example, an analyte sensor 1120 can include a glucose sensing probe configured to pierce the surface of the skin 1121. In some examples, a disease management system 1101 may include a plurality of analyte sensors 1120 to detect one or more analytes. In some examples, an analyte sensor 1120 may be configured to detect a plurality of analytes. Sensed analytes may include, but are not limited to, glucose, insulin, and other analytes. An analyte sensor 1120 may be configured to communicate with an analyte detector 1126. The analyte detector 1126 may be configured to receive a signal of one or more analyte sensors 1120 in order to measure one or more analytes in the blood of the patient. The analyte detector 1126 may be configured to communicate with the controller 1138. For example, the analyte detector 1126 may be configured to, for example, send analyte values to the controller 1138 and receive control signals from the controller.

A disease management system 1101 may include a medication catheter 1122. The medication catheter 1122 may be configured to administer medication, including, but not limited to insulin, to the patient. The medication catheter 1122 may receive medication from a medication bladder 1128 configured to contain medication to be administered. The medication bladder 1128 may be configured to contain medication for a prolonged period, such as 1 day, 3 days, 6 days, or more. The medication bladder 1128 may be configured to contain certain medication types, such as insulin. In some examples, a disease management system 1101 may include a plurality of medication bladders 1128 for one or more reservoirs of the same or different medications. In some examples, a disease management system 1101 may be configured to mix medications from medication bladders 1128 prior to administration to the patient. A pump 1130 may be configured to cause medication to be administered from the bladder 1128 to the patient through the insulin catheter 1122. A pump 1130 may include, but is not limited to, a pump such as described herein.

A disease management system 1101 may optionally include a physiological sensor 1124. The physiological sensor 1124 may include a pulse rate sensor, temperature sensor, pulse oximeter, the like or a combination thereof. In some examples, a disease management system 1101 may be configured to include a plurality of physiological sensors. The physiological sensor 1124 may be configured to communicate with a physiological detector 1134. The physiological detector 1134 may be configured to receive a signals of the physiological sensor 1124. The physiological detector 1134 may be configured to measure or determine and communicate a physiological value from the signal. The physiological detector 1134 may be configured to communicate with the controller 1138. For example, the physiological detector 1134 may be configured to, for example, send measured physiological values to the controller 1138 and receive control signals from the controller.

A disease management system 1101 may include one or more local user interfacing components 1136. For example, a local user interfacing component 1136 may include, but is not limited to one or more optical displays, haptic motors, audio speakers, and user input detectors. In some examples, an optical display may include an LED light configured to display a plurality of colors. In some examples, an optical display may include a digital display of information associated with the disease management system 1101, including, but not limited to, device status, medication status, patient status, measured analyte or physiological values, the like or a combination thereof. In some examples, a user input detector may include an inertial measurement unit, tap detector, touch display, or other component configured to accept and receive user input. In some examples, audio speakers may be configured to communicate audible alarms related to device status, medication status user status, the like or a combination thereof. A controller 1138 may be configured to communicate with the one or more local interfacing components 1136 by, for example, receiving user input from the one or more user input components or sending control signals to, for example, activate a haptic motor, generate an output to the optical display, generate an audible output, or otherwise control one or more of the local user interfacing components 1136.

A disease management system 1101 may include one or more communication components 1140. A communication component 1140 can include, but is not limited to one or more radios configured to emit Bluetooth, cellular, Wi-Fi, or other wireless signals. In some examples, a communication component 1140 can include a port for a wired connection.

Additionally, a disease management system 1101 may include an NFC tag 1142 to facilitate in communicating with one or more hardware processors. The one or more communication components 1140 and NFC tag 1142 may be configured to communicate with the controller 1138 in order to send and/or receive information associated with the disease management system 1101. For example, a controller 1138 may communicate medication information and measured values through the one or more communication components 1140 to an external device. Additionally, the controller 1138 may receive instructions associated with measurement sampling rates, medication delivery, or other information associated with operation of the management system 1101 through the one or more communication components 1140 from one or more external devices.

A disease management system 1101 may include one or more power components 1144. The power components may include, but are not limited to one or more batteries and power management components, such as a voltage regulator. Power from the one or more power components 1144 may be accessed by the controller and/or other components of the disease management system 1101 to operate the disease management system 1101.

A disease management system 1101 may have one or more power and sleep modes to help regulate power usage. For example, a disease management system 1101 may have a sleep mode. The sleep mode may be a very low power mode with minimal functions, such as the RTC (or real time clock) and alarms to wake the system and take a temperature measurement of the system, or the like. In another example, a disease management system 1101 may include a measure temperature mode which may correspond to a low power mode with reduced functions. The measure temperature mode may be triggered by the RTC where the system is configured to take a temperature measurement, save the value, and return the system to a sleep mode. In another example, a disease management system 1101 may include a wake up mode. The wake up mode may be triggered by an NFC device and allow the system to pair with an external device with, for example, Bluetooth. If a pairing event does not occur, the system may return to sleep mode. In another example, a disease management system 1101 may include a pairing mode. The pairing mode may be triggered by an NFC device. When a controlling application is recognized, the system may proceed to pair with the application and set the system to an on condition and communicate to the cloud or other external device to establish initial data movement. In another example, a disease management system 1101 may include a rest mode where the system is configured to enter a lower power mode between measurements. In another example, a disease management system 1101 may include a data acquisition mode where the system is configured to enter a medium power mode where data acquisition takes place. In another example, a disease management system 1101 may include a parameter calculation mode where the system is configured to enter a medium power mode where parameter calculations, such as a blood glucose calculations, are performed and data is communicated to an external device and/or the cloud. In another example, a disease management system 1101 may include a pump mode where the system is configured to enter a higher power mode where the pump draws power to deliver medication to the patient.

A disease management system 1101 may include one or more connector test points 1146. The connecter test points may be configured to aid in programming, debugging, testing or other accessing of the disease management system 1101. In some examples, connector test points 1146 may include, for example, a GPIO spare, UART receiver or transmitter, the like or a combination thereof.

FIG. 6 illustrates an example implementation of a disease management system 1103 and applicator 1190 for applying a disease management system 1103 to a patient. Disease management system 1103 can include any one or more of the features discussed above with respect to the disease management system 1101 in addition to the features described below. In the illustrated example, an applicator 1190 may be configured to mate with the disease management system 1103. In some examples, an applicator 1190 may include a safety button 1192 for release or other interaction with the applicator 1190. In the illustrated example, a disease management system 1103 may include one or more LEDs 1160 that may be configured to output information using one or more of color, frequency, and length of display. In some examples, the disease management system 1103 may include a buzzer 1176, haptic actuator 1170, or other feedback mechanism, such as a speaker to output information to the patient, such as an alarm. In some examples, a disease management system 1103 may include a battery 1174, controller 1172. In some examples, a disease management system 1103 may include aspects of a medication administration system (e.g. an insulin administration system), such as a bladder 1180, a bladder pressure applicator 1178 to provide pressure on the bladder (such as a component of a pump), actuator 1182, pump gears 1184, and a pump 1186. In some examples, a disease management system 1103 may include one or more needles 1158 that may include one or more analyte sensors (such as a glucose sensor described herein) 1156. In some examples, a disease management system 1103 may include one or more needles 1162 that may include one or more cannulas 1164 configured to administer medication to the patient (e.g., an insulin formulation described herein). In some examples, a disease management system 1103 may include an air bubble sensor 1152 configured to detect the presence of air bubbles in the medication prior to delivery to the patient. In some examples, a glucose control system 1103 may include one or more physiological sensors 1154, such as a non-invasive physiological sensor including but not limited to a pulse sensor. In some examples, the disease management system 1103 may include a base plate 1106 and an adhesive layer 1168 below the base plate 1106 to provide adhesion of the disease management system 1103 to the patient's skin. As described below, a housing of the disease management system 1103 may consist of a combination of flexible and rigid

material so as to both provide support for the components of the disease management system 1103 and allow conforming, at least in part, of the disease management system 1103 to the skin of the patient.

The adhesive layer 1168 may be configured to provide adhesion for a prolonged period. For example, the adhesive layer 1168 may be configured to adhere the disease management system 1103 to the skin of a patient for a period of 1 day, 3 days, 6 days, or more or fewer days or hours. In some examples, the adhesive layer may be configured to have an adhesive force sufficient to prevent accidental removal or movement of the disease management system 1103 during the intended period of use of the disease management system 1103. In some examples, the adhesive layer 1168 may be a single layer of adhesive across at least a portion of a surface the disease management system 1103 that is configured to interface with the patient. In some examples, the adhesive layer 1168 may include a plurality of adhesive areas on a surface of the disease management system 1103 that is configured to interface with the patient. In some examples, the adhesive layer 1168 may be configured to be breathable, adhere to the patient's skin after wetting by humidity or liquids such as tap water, saltwater, and chlorinated water. A thickness of the adhesive may be, for example, in a range of 0.1 to 0.5 mm or in a range of more or less thickness.

In some examples, a needle 1158, 1162 may be inserted at different depths based on a patient age, weight, or other parameter. For example, a depth of insertion of a medication cannula may be approximately 3 mm for 7 to 12 year olds. In another example, a depth of insertion of a medication cannula may be approximately 4 mm for 13 year olds and older. In another example, a depth of insertion of a medication needle may be approximately 4 to 4.5 mm for 7 to 12 year olds. In another example, a depth of insertion of a medication needle may be approximately 5 to 5.5 mm for 13 year olds and older. In another example, a depth of insertion of an analyte sensor may be approximately 3 mm for 7 to 12 year olds. In another example, a depth of insertion of an analyte sensor may be approximately 4 mm for 13 year olds and older. In another example, a depth of insertion for a needle associated with an analyte sensor may be approximately 4 to 4.5 mm for 7 to 12 year olds. In another example, a depth of insertion for a needle associated with an analyte sensor may be approximately 5 to 5.5 mm for 13 year olds and older. However, other values or ranges for any of the inserted components are also possible.

While the above detailed description has shown, described, and pointed out novel features, it can be understood that various omissions, substitutions, and changes in the form and details of the devices or algorithms illustrated can be made without departing from the the disclosure. As can be recognized, certain portions of the description herein can be embodied within a form that does not provide all of the features and benefits set forth herein, as some features can be used or practiced separately from others. The invention is defined by the appended claims.

## Claims

**1.** A glucose monitoring device comprising:

a reference electrode (RE);
a working electrode (WE), wherein the working electrode (WE) is disposed in the vicinity of the reference electrode (RE);
an enzymatic layer (70) comprising glucose oxidase, wherein the glucose oxidase is capable of catalyzing a reaction of glucose and oxygen to generate one or more oxidized species;
a first permeability-selective layer (40) for reducing or blocking the diffusion of glucose to the enzymatic layer (70);
an oxygen-replenishing layer (50) comprising one or more enzymes, wherein at least one enzyme in the oxygen-replenishing layer (50) is capable of consuming at least one oxidized species from the enzymatic layer (70) and generating oxygen; and
an outer protective layer (60); wherein
the enzymatic layer (70) is in closer proximity to the working electrode (WE) than the oxygen-replenishing layer (50), and wherein the loading of the glucose oxidase in the enzymatic layer (70) and the loading of the at least one oxygen generating enzymes in the oxygen-replenishing layer (50) result in the rate of reaction of the glucose oxidase in the enzymatic layer (70) and the rate of reaction of the oxygen-generating enzyme in the oxygen-replenishing layer (50) to be substantially the same such that the glucose monitoring device is temperature independent within an operating temperature range.

**2.** The glucose monitoring device of claim 1, wherein the glucose oxidase is present in a matrix comprising bovine serum albumin (BSA), poly-ortho-phenylenediamine (PoPD), poly-meta-phenylenediamine (PmPD), poly-para-phenylenediamine (PpPD), polypyrrole, polyaniline, or combinations thereof.

**3.** The glucose monitoring device of claim 1, wherein the glucose oxidase is present in a hydrogel matrix, preferably wherein the hydrogel matrix comprises one or more materials selected from the group consisting of cellulose acetate,

chitosan, poly(2-hydroxyethyl methacrylate)(pHEMA), polyethylene glycol diamine, 3,6,9-Trioxaundecanedioic acid, sodium citrate, polyvinyl alcohol and polyethylenimine(PEI), and combinations thereof, more preferably wherein the hydrogel matrix comprises two or more crosslinked materials.

**4.** The glucose monitoring device of claim 3, wherein

the hydrogel matrix further comprises one or more polymeric materials that render the hydrogel matrix with a negative charge, preferably wherein the one or more polymeric materials comprise poly(sodium 4-styrenesulfonate), poly(4-styrenesulfonic acid-co-maleic acid) sodium salt, poly(acrylic acid-co-maleic acid), or poly(vinylsulfonic acid) sodium salt, or combinations thereof; and/or wherein
the glucose oxidase is cross-linked; and/or wherein
the oxidized species of the reaction of glucose and oxygen in the enzymatic layer (70) comprises hydrogen peroxide ($H_2O_2$) and gluconolactone; and/or wherein
the oxygen-replenishing layer (50) comprises one or more enzymes selected from the group consisting of peroxidases, transferases, hydrolases, oxidases, kinases, superoxidases, phosphatases, pyrophosphatases, hydroxylases, dioxygenases, dehydrogenases, carboxylases, aminases, catalases, phosphohydrolases, diaminases, reductases, synthases, and caspases, and combinations thereof, preferably wherein the oxygen-replenishing layer (50) comprises catalase; and/or wherein
the oxygen-replenishing layer (50) further comprises a glucokinase or a gluconate.

**5.** The glucose monitoring device of claim 4, wherein the oxygen-replenishing layer (50) further comprises an ascorbate peroxidase or ascorbate oxidase.

**6.** The glucose monitoring device of any one of claims 1 to 5, wherein either or both of the enzymatic layer (70) and the oxygen-replenishing layer (50) further comprises one or more oxygen binding proteins or oxygen binding globins, or combinations thereof, preferably wherein the oxygen binding protein comprises hemerythrin, or wherein the oxygen binding globin comprises myoglobin, hemoglobin, or a combination thereof.

**7.** The glucose monitoring device of any one of claims 1 to 6, wherein the first permeability-selective layer (40) comprises one or more polymers selected from the group consisting of a polyacetal, a polyolefin, a polyacrylic, a polycarbonate, a polystyrene, a polyester, a polyamide, polyamideimides, a polyarylate, a polyarylsulfone, a polyethersulfone, a polyphenylene sulfide, a polyvinyl chloride, a polyethylene oxide, a polysulfone, a polyimide, a polyetherimide, a polytetrafluoroethylene, a polyetherketone, a polyether etherketone, a polyether ketone ketone, a polybenzoxazole, a polyphthalide, a polyacetal, a polyanhydride, a polyvinyl ether, a polyvinyl thioether, a polyvinyl alcohol, a polyvinyl ketone, a polyvinyl halide, a polyvinyl nitrile, a polyvinyl ester, a polysulfonate, a polysulfide, a poly(allyl amine), a polythioester, a polysulfone, a polysulfonamide, a polyurea, a polyphosphazene, a polysilazane, a polyvinylchloride, a polyvinyl acetate, a humic acid, a cellulose acetate, a polythiophene, a polyphenylene diamine, a polypyrrole, a polynaphthalene a polyurethane, an ethylene propylene diene rubber, a polytetrafluoroethylene, a fluorinated ethylene propylene, a sulfonated tetrafluoroethylene based fluoropolymer-copolymer, a perfluoroalkoxyethylene, a polychlorotrifluoroethylene, a polyvinylidene fluoride, and a polysiloxane, and combinations thereof, preferably wherein the first permeability-selective layer (40) comprises poly(ortho-phenylenediamine) (PoPD), poly(meta-phenylenediamine) (PmPD), or poly(para-phenylenediamine) (PpPD), or combinations thereof.

**8.** The glucose monitoring device of any one of claims 1 to 7, wherein the first permeability-selective layer (40) is disposed between the enzymatic layer (70) and the oxygen-replenishing layer (50), preferably wherein the first permeability-selective layer (40) is in direct contact with one or both of the enzymatic layer (70) and the oxygen-replenishing layer (50).

**9.** The glucose monitoring device of any one of claims 1 to 7, wherein the first permeability-selective layer (40) is disposed between the oxygen-replenishing layer (50) and the outer protective layer (60), preferably wherein the first permeability-selective layer (40) is in direct contact with one or both of oxygen-replenishing layer (50) and the outer protective layer (60).

**10.** The glucose monitoring device of any one of claims 1 to 9, further comprising a second permeability-selective layer for blocking the contact of one or more redox active species with the working electrode (WE) and/or the reference electrode (RE), preferably wherein the second permeability-selective layer is disposed between the working electrode (WE) and the enzymatic layer (70), more preferably wherein the second permeability-selective layer is in direct contact with one or both of the working electrode (WE) and the enzymatic layer (70).

**11.** The glucose monitoring device of claim 10, wherein the second permeability-selective layer comprises electropolymerized PoPD, electropolymerized PmPD, electropolymerized PpPD, diamino-naphthalene (DAN), amino naphthol, polypyrrole, polyaniline, cellulose acetate, or an ionic polymer, or combinations thereof.

**12.** The glucose monitoring device of claim 10 or 11, wherein the second permeability-selective layer has a thickness from about 1 nm to about 10 μm, preferably wherein the second permeability-selective layer has a thickness of about 10 nm to about 300 nm.

**13.** The glucose monitoring device of any one of claims 10 to 12, wherein the one or more redox active species comprise endogenous or exogenous compounds present in a mammal's bodily fluid, tissue fluid, or serum, preferably wherein the one or more redox active species comprise ascorbic acid, uric acid, or acetaminophen, or combinations thereof.

**14.** The glucose monitoring device of any one of claims 1 to 13, wherein the outer protective layer (60) comprises a polymer, a hydrogel, or a combination thereof for reducing or inhibiting protein adhesion, preferably wherein the outer protective layer (60) comprises polyvinyl alcohol (PVA), Nafion™, or a combination thereof.

**15.** The glucose monitoring device of any one of claims 10 to 14, wherein the second permeability-selective layer is disposed between the working electrode (WE) and the enzymatic layer (70), the enzymatic layer (70) is disposed between the second permeability-selective layer and the first permeability-selective layer (40), the first permeability-selective layer (40) is disposed between the enzymatic layer (70) and the oxygen-replenishing layer (50), and the oxygen-replenishing layer (50) is disposed between the first permeability-selective layer (40) and the outer protective layer (60).

## Patentansprüche

**1.** Glukoseüberwachungsvorrichtung, aufweisend:

eine Referenzelektrode (RE);
eine Arbeitselektrode (WE), wobei die Arbeitselektrode (WE) in der Nähe der Referenzelektrode (RE) angeordnet ist;
eine enzymatische Schicht (70), die Glukoseoxidase aufweist, wobei die Glukoseoxidase in der Lage ist, eine Reaktion von Glukose und Sauerstoff zu katalysieren, um eine oder mehrere oxidierte Spezies zu erzeugen;
eine erste permselektive Schicht (40) zum Vermindern oder Blockieren der Diffusion von Glukose zur enzymatischen Schicht (70);
eine Sauerstoffergänzungsschicht (50), die ein oder mehrere Enzyme aufweist, wobei mindestens ein Enzym in der Sauerstoffergänzungsschicht (50) in der Lage ist, mindestens eine oxidierte Spezies aus der enzymatischen Schicht (70) zu verbrauchen und Sauerstoff zu erzeugen; und
eine äußere Schutzschicht (60),
wobei die enzymatische Schicht (70) näher an der Arbeitselektrode (WE) liegt als die Sauerstoffergänzungsschicht (50), und wobei die Beladung der Glukoseoxidase in der enzymatischen Schicht (70) und die Beladung des mindestens einen sauerstofferzeugenden Enzyms in der Sauerstoffergänzungsschicht (50) dazu führen, dass die Reaktionsrate der Glukoseoxidase in der enzymatischen Schicht (70) und die Reaktionsrate des sauerstofferzeugenden Enzyms in der Sauerstoffergänzungsschicht (50) im Wesentlichen gleich sind, so dass die Glukoseüberwachungsvorrichtung innerhalb eines Betriebstemperaturbereichs temperaturunabhängig ist.

**2.** Glukoseüberwachungsvorrichtung nach Anspruch 1, wobei die Glukoseoxidase in einer Matrix vorhanden ist, die Rinderserumalbumin (BSA), Poly-ortho-phenylendiamin (PoPD), Polymetaphenylendiamin (PmPD), Poly-paraphenylendiamin (PpPD), Polypyrrol, Polyanilin oder Kombinationen davon aufweist.

**3.** Glukoseüberwachungsvorrichtung nach Anspruch 1, wobei
die Glukoseoxidase in einer Hydrogelmatrix vorliegt, wobei vorzugsweise die Hydrogelmatrix ein oder mehrere Materialien aufweist, die ausgewählt sind aus der Gruppe bestehend aus Celluloseacetat, Chitosan, Poly(2-hydroxyethylmethacrylat) (pHEMA), Polyethylenglykoldiamin, 3,6,9-Trioxaundecandisäure, Natriumcitrat, Polyvinylalkohol und Polyethylenimin (PEI) und Kombinationen davon, wobei die Hydrogelmatrix vorzugsweise zwei oder mehr vernetzte Materialien aufweist.

**4.** Glukoseüberwachungsvorrichtung nach Anspruch 3, wobei

die Hydrogelmatrix ferner ein oder mehrere polymere Materialien aufweist, die der Hydrogelmatrix eine negative Ladung verleihen, wobei vorzugsweise das eine oder die mehreren polymeren Materialien Poly(natrium-4-styrolsulfonat), Poly(4-styrolsulfonsäure-co-maleinsäure)-Natriumsalz, Poly(acrylsäure-co-maleinsäure) oder Poly(vinylsulfonsäure)-Natriumsalz oder Kombinationen davon aufweisen, und/oder
wobei die Glukoseoxidase vernetzt ist, und/oder
wobei die oxidierten Spezies der Reaktion von Glukose und Sauerstoff in der enzymatischen Schicht (70) Wasserstoffperoxid ($H_2O_2$) und Gluconolacton aufweisen, und/oder
wobei die Sauerstoffergänzungsschicht (50) ein oder mehrere Enzyme aufweist, die ausgewählt sind aus der Gruppe bestehend aus Peroxidasen, Transferasen, Hydrolasen, Oxidasen, Kinasen, Superoxidasen, Phosphatasen, Pyrophosphatasen, Hydroxylasen, Dioxygenasen, Dehydrogenasen, Carboxylasen, Aminasen, Katalasen, Phosphohydrolasen, Diaminasen, Reduktasen, Synthasen und Caspasen und Kombinationen davon, wobei vorzugsweise die Sauerstoffergänzungsschicht (50) Katalase aufweist, und/oder
wobei die Sauerstoffergänzungsschicht (50) ferner eine Glukokinase oder ein Gluconat aufweist.

**5.** Glukoseüberwachungsvorrichtung nach Anspruch 4, wobei die Sauerstoffergänzungsschicht (50) ferner eine Ascorbatperoxidase oder Ascorbatoxidase aufweist.

**6.** Glukoseüberwachungsvorrichtung nach einem der Ansprüche 1 bis 5, wobei die enzymatische Schicht (70) und/oder die Sauerstoffergänzungsschicht (50) ferner eine oder mehrere Sauerstoff bindende Proteine oder Sauerstoff bindende Globine oder Kombinationen davon aufweisen, wobei vorzugsweise das Sauerstoff bindende Protein Hämerythrin aufweist, oder wobei das Sauerstoff bindende Globin Myoglobin, Hämoglobin oder eine Kombination davon aufweist.

**7.** Glukoseüberwachungsvorrichtung nach einem der Ansprüche 1 bis 6, wobei die erste permselektive Schicht (40) ein oder mehrere Polymere aufweist, die ausgewählt sind aus der Gruppe bestehend aus einem Polyacetal, einem Polyolefin, einem Polyacryl, einem Polycarbonat, einem Polystyrol, einem Polyester, einem Polyamid, Polyamidimiden, einem Polyarylat, einem Polyarylsulfon, einem Polyethersulfon, einem Polyphenylensulfid, einem Polyvinylchlorid, einem Polyethylenoxid, einem Polysulfon, einem Polyimid, einem Polyetherimid, einem Polytetrafluorethylen, einem Polyetherketon, einem Polyetheretherketon, einem Polyetherketonketon, einem Polybenzoxazol, einem Polyphthalid, einem Polyacetal, einem Polyanhydrid, einem Polyvinylether, einem Polyvinylthioether, einem Polyvinylalkohol, einem Polyvinylketon, einem Polyvinylhalogenid, einem Polyvinylnitril, einem Polyvinylester, einem Polysulfonat, einem Polysulfid, einem Poly(allylamin), einem Polythioester, einem Polysulfon, einem Polysulfonamid, einem Polyharnstoff, einem Polyphosphazen, einem Polysilazan, einem Polyvinylchlorid, einem Polyvinylacetat, einer Huminsäure, einem Celluloseacetat, einem Polythiophen, einem Polyphenylendiamin, einem Polypyrrol, einem Polynaphthalin, einem Polyurethan, einem Ethylen-Propylen-Dien-Kautschuk, einem Polytetrafluorethylen, einem fluorierten Ethylenpropylen, einem sulfonierten Fluorpolymer-Copolymer auf Tetrafluorethylenbasis, einem Perfluoralkoxyethylen, einem Polychlortrifluorethylen, einem Polyvinylidenfluorid und einem Polysiloxan und Kombinationen davon, wobei vorzugsweise die erste permselektive Schicht (40) Poly(ortho-phenylendiamin) (PoPD), Poly(meta-phenylendiamin) (PmPD) oder Poly(para-phenylendiamin) (PpPD) oder Kombinationen davon aufweist.

**8.** Glukoseüberwachungsvorrichtung nach einem der Ansprüche 1 bis 7, wobei die erste permselektive Schicht (40) zwischen der enzymatischen Schicht (70) und der Sauerstoffergänzungsschicht (50) angeordnet ist, wobei vorzugsweise die erste permselektive Schicht (40) in direktem Kontakt mit der enzymatischen Schicht (70) und/oder der Sauerstoffergänzungsschicht (50) steht.

**9.** Glukoseüberwachungsvorrichtung nach einem der Ansprüche 1 bis 7, wobei die erste permselektive Schicht (40) zwischen der Sauerstoffergänzungsschicht (50) und der äußeren Schutzschicht (60) angeordnet ist, wobei vorzugsweise die erste permselektive Schicht (40) in direktem Kontakt mit der Sauerstoffergänzungsschicht (50) und/oder der äußeren Schutzschicht (60) steht (60).

**10.** Glukoseüberwachungsvorrichtung nach einem der Ansprüche 1 bis 9, ferner aufweisend eine zweite permselektive Schicht zum Blockieren des Kontakts einer oder mehrerer redoxaktiver Spezies mit der Arbeitselektrode (WE) und/oder der Referenzelektrode (RE), wobei die zweite permselektive Schicht vorzugsweise zwischen der Arbeitselektrode (WE) und der enzymatischen Schicht (70) angeordnet ist, wobei die zweite permselektive Schicht vorzugsweise in direktem Kontakt mit der Arbeitselektrode (WE) und/oder der enzymatischen Schicht (70) steht.

**11.** Glukoseüberwachungsvorrichtung nach Anspruch 10, wobei die zweite permselektive Schicht elektropolymerisiertes PoPD, elektropolymerisiertes PmPD, elektropolymerisiertes PpPD, Diaminonaphthalin (DAN), Aminonaphthol,

Polypyrrol, Polyanilin, Celluloseacetat oder ein ionisches Polymer oder Kombinationen davon aufweist.

**12.** Glukoseüberwachungsvorrichtung nach Anspruch 10 oder 11, wobei die zweite permselektive Schicht eine Dicke von etwa 1 nm bis etwa 10 μm aufweist, wobei vorzugsweise die zweite permselektive Schicht eine Dicke von etwa 10 nm bis etwa 300 nm aufweist.

**13.** Glukoseüberwachungsvorrichtung nach einem der Ansprüche 10 bis 12, wobei die eine oder die mehreren redoxaktiven Spezies endogene oder exogene Verbindungen aufweisen, die in der Körperflüssigkeit, Gewebeflüssigkeit oder dem Serum eines Säugetiers vorhanden sind, wobei vorzugsweise die eine oder die mehreren redoxaktiven Spezies Ascorbinsäure, Harnsäure oder Acetaminophen oder Kombinationen davon aufweisen.

**14.** Glukoseüberwachungsvorrichtung nach einem der Ansprüche 1 bis 13, wobei die äußere Schutzschicht (60) ein Polymer, ein Hydrogel oder eine Kombination davon aufweist, um die Proteinadhäsion zu vermindern oder zu hemmen, und wobei die äußere Schutzschicht (60) vorzugsweise Polyvinylalkohol (PVA), Nafion™ oder eine Kombination davon aufweist.

**15.** Glukoseüberwachungsvorrichtung nach einem der Ansprüche 10 bis 14, wobei die zweite permselektive Schicht zwischen der Arbeitselektrode (WE) und der enzymatischen Schicht (70) angeordnet ist, die enzymatische Schicht (70) zwischen der zweiten permselektiven Schicht und der ersten permselektiven Schicht (40) angeordnet ist, die erste permselektive Schicht (40) zwischen der enzymatischen Schicht (70) und der Sauerstoffergänzungsschicht (50) angeordnet ist und die Sauerstoffergänzungsschicht (50) zwischen der ersten permselektiven Schicht (40) und der äußeren Schutzschicht (60) angeordnet ist.

## Revendications

**1.** Dispositif de surveillance de glucose comprenant :

une électrode de référence (RE) ;
une électrode de travail (WE), dans lequel l'électrode de travail (WE) est disposée dans les environs de l'électrode de référence (RE) ;
une couche enzymatique (70) comprenant de la glucose oxydase, dans lequel la glucose oxydase est capable de catalyser une réaction du glucose et de l'oxygène pour générer une ou plusieurs espèces oxydées ;
une première couche sélective de perméabilité (40) pour réduire ou bloquer la diffusion du glucose à la couche enzymatique (70) ;
une couche de recharge en oxygène (50) comprenant une ou plusieurs enzymes, dans lequel au moins une enzyme dans la couche de recharge en oxygène (50) est capable de consommer au moins une espèce oxydée depuis la couche enzymatique (70) et de générer de l'oxygène ; et
une couche protectrice externe (60) ; dans lequel
la couche enzymatique (70) est plus proche de l'électrode de travail (WE) que la couche de recharge en oxygène (50), et dans lequel le chargement de la glucose oxydase dans la couche enzymatique (70) et le chargement de l'au moins une enzyme générant de l'oxygène dans la couche de recharge en oxygène (50) résultent en ce que la vitesse de réaction de la glucose oxydase dans la couche enzymatique (70) et la vitesse de réaction de l'enzyme générant de l'oxygène dans la couche de recharge en oxygène (50) sont essentiellement identiques de telle sorte que le dispositif de surveillance de glucose est indépendant de la température au sein d'une plage de température de fonctionnement.

**2.** Dispositif de surveillance de glucose selon la revendication 1, dans lequel la glucose oxydase est présente dans une matrice comprenant de l'albumine de sérum bovin (BSA), de la poly-ortho-phénylènediamine (PoPD), de la poly-méta-phénylènediamine (PmPD), de la poly-para-phénylènediamine (PpPD), du polypyrrole, de la polyaniline, ou des combinaison de ceux-ci.

**3.** Dispositif de surveillance de glucose selon la revendication 1, dans lequel la glucose oxydase est présente dans une matrice d'hydrogel, de préférence dans lequel la matrice d'hydrogel comprend une ou plusieurs matières choisies dans le groupe constitué par l'acétate de cellulose, le chitosane, le poly(2-hydroxyéthylméthacrylate)(pHEMA), la polyéthylène glycol diamine, l'acide 3,6,9-trioxaundécanedioïque, le citrate de sodium, l'alcool polyvinylique et la polyéthylèneimine (PEI), et les combinaisons de ceux-ci, plus préférentiellement dans lequel la matrice d'hydrogel comprend deux matières réticulées ou plus.

4. Dispositif de surveillance de glucose selon la revendication 3, dans lequel

la matrice d'hydrogel comprend en outre une ou plusieurs matières polymères qui confèrent à la matrice d'hydrogène une charge négative, de préférence dans lequel la ou les matières polymères comprennent le poly(4-styrènesulfonate de sodium), le sel de sodium du poly(acide 4-styrènesulfonique-co-acide maléique), le poly(acide acrylique-co-acide maléique), ou le sel de sodium du poly(acide vinylsulfonique), ou des combinaisons de ceux-ci ; et/ou dans lequel
la glucose oxydase est réticulée ; et/ou dans lequel
l'espèce oxydée de la réaction du glucose et de l'oxygène dans la couche enzymatique (70) comprend du peroxyde d'hydrogène (H202) et de la gluconolactone ; et/ou dans lequel
la couche de recharge en oxygène (50) comprend une ou plusieurs enzymes choisies dans le groupe constitué par les peroxydases, les transférases, les hydrolases, les oxydases, les kinases, les superoxydases, les phosphatases, les pyrophosphatases, les hydroxylases, les dioxygénases, les déshydrogénases, les carboxylases, les aminases, les catalases, les phosphohydrolases, les diaminases, les réductases, les synthases et les caspases, et des combinaisons de ceux-ci, préférentiellement dans lequel la couche de recharge en oxygène (50) comprend de la catalase ; et/ou dans lequel
la couche de recharge en oxygène (50) comprend en outre une glucokinase ou un gluconate.

5. Dispositif de surveillance de glucose selon la revendication 4, dans lequel la couche de recharge en oxygène (50) comprend en outre une ascorbate peroxydase ou une ascorbate oxydase.

6. Dispositif de surveillance de glucose selon l'une quelconque des revendications 1 à 5, dans lequel l'une ou l'autre parmi la couche enzymatique (70) et la couche de recharge en oxygène (50), ou les deux, comprennent en outre une ou plusieurs protéines se liant à l'oxygène ou globines se liant à l'oxygène, ou des combinaisons de celle-ci, préférentiellement dans lequel la protéine se liant à l'oxygène comprend l'hémérythrine, ou dans lequel la globine se liant à l'oxygène comprend la myoglobine, l'hémoglobine ou une combinaison de celles-ci.

7. Dispositif de surveillance de glucose selon l'une quelconque des revendications 1 à 6, dans lequel la première couche sélective de perméabilité (40) comprend un ou plusieurs polymères choisis dans le groupe constitué par un polyacétal, une polyoléfine, un polyacrylate, un polycarbonate, un polystyrène, un polyester, un polyamide, des polyamideimides, un polyarylate, une polyarylsulfone, une polyéthersulfone, un polysulfure de phénylène, un polychlorure de vinyle, un polyoxyde d'éthylène, une polysulfone, un polyimide, un polyétherimide, un polytétrafluoroéthylène, une polyéthercétone, une polyéther éthercétone, une polyéther cétone cétone, un polybenzoxazole, un polyphtalide, un polyacétal, un polyanhydride, un polyéther de vinyle, un polythioéther de vinyle, un alcool polyvinylique, une polyvinylcétone, un polyhalogénure de vinyle, un polyvinylnitrile, un polyester de vinyle, un polysulfonate, un polysulfure, une poly(allylamine), un polythioester, une polysulfone, un polysulfonamide, une polyurée, un polyphosphazène, un polysilazane, un polychlorure de vinyle, un polyacétate de vinyle, un acide humique, un acétate de cellulose, un polythiophène, une polyphénylène diamine, un polypyrrole, un polynaphtalène, un polyuréthane, un caoutchouc éthylène propylène diène, un polytétrafluoroéthylène, un éthylène propylène fluoré, un copolymère de fluoropolymère à base de tétrafluoroéthylène sulfoné, un perfluoroalkoxyéthylène, un polychlorotrifluoroéthylène, un polyfluorure de vinylidène et un polysiloxane, et des combinaisons de ceux-ci, de préférence dans lequel la première couche sélective de perméabilité (40) comprend de la poly(ortho-phénylènediamine) (PoPD), de la poly(méta-phénylènediamine) (PmPD) ou de la poly(para-phénylènediamine) (PpPD), ou des combinaisons de celles-ci.

8. Dispositif de surveillance de glucose selon l'une quelconque des revendications 1 à 7, dans lequel la première couche sélective de perméabilité (40) est disposée entre la couche enzymatique (70) et la couche de recharge en oxygène (50), de préférence dans lequel la première couche sélective de perméabilité (40) est en contact direct avec une parmi la couche enzymatique (70) et la couche de recharge en oxygène (50), ou les deux.

9. Dispositif de surveillance de glucose selon l'une quelconque des revendications 1 à 7, dans lequel la première couche sélective de perméabilité (40) est disposée entre la couche de recharge en oxygène (50) et la couche protectrice externe (60), de préférence dans lequel la première couche sélective de perméabilité (40) est en contact direct avec une parmi la couche de recharge en oxygène (50) et la couche protectrice externe (60), ou les deux.

10. Dispositif de surveillance de glucose selon l'une quelconque des revendications 1 à 9, comprenant en outre une seconde couche sélective de perméabilité pour bloquer le contact d'une ou de plusieurs espèces redox actives avec l'électrode de travail (WE) et/ou l'électrode de référence (RE), de préférence dans lequel la seconde couche sélective

de perméabilité est disposée entre l'électrode de travail (WE) et la couche enzymatique (70), plus préférentiellement dans lequel la seconde couche sélective de perméabilité est en contact direct avec une parmi l'électrode de travail (WE) et la couche enzymatique (70), ou les deux.

**11.** Dispositif de surveillance de glucose selon la revendication 10, dans lequel la seconde couche sélective de perméabilité comprend de la PoPD électropolymérisée, de la PmPD électropolymérisée, de la PpPD électropolymérisée, du diamino-naphtalène (DAN), de l'aminonaphtol, du polypyrrole, de la polyaniline, de l'acétate de cellulose ou un polymère ionique, des combinaisons de ceux-ci.

**12.** Dispositif de surveillance de glucose selon la revendication 10 ou 11, dans lequel la seconde couche sélective de perméabilité a une épaisseur d'environ 1 nm à environ 10 μm, de préférence dans lequel la seconde couche sélective de perméabilité a une épaisseur d'environ 10 nm à environ 300 nm.

**13.** Dispositif de surveillance de glucose selon l'une quelconque des revendications 10 à 12, dans lequel la ou les espèces redox actives comprennent des composés endogènes ou exogènes présents dans un fluide corporel, un fluide tissulaire ou un sérum d'un mammifère, de préférence dans lequel la ou les espèces redox actives comprennent l'acide ascorbique, l'acide urique ou l'acétaminophène, ou des combinaisons de ceux-ci.

**14.** Dispositif de surveillance de glucose selon l'une quelconque des revendications 1 à 13, dans lequel la couche protectrice externe (60) comprend un polymère, un hydrogel, ou une combinaison de ceux-ci pour réduire ou inhiber l'adhérence des protéines, de préférence dans lequel la couche protectrice externe (60) comprend de l'alcool polyvinylique (PVA), du Nafion™, ou une combinaison de ceux-ci.

**15.** Dispositif de surveillance de glucose selon l'une quelconque des revendications 10 à 14, dans lequel la seconde couche sélective de perméabilité est disposée entre l'électrode de travail (WE) et la couche enzymatique (70), la couche enzymatique (70) est disposée entre la seconde couche sélective de perméabilité et la première couche sélective de perméabilité (40), la première couche sélective de perméabilité (40) est disposée entre la couche enzymatique (70) et la couche de recharge en oxygène (50), et la couche de recharge en oxygène (50) est disposée entre la première couche sélective de perméabilité (40) et la couche protectrice externe (60).

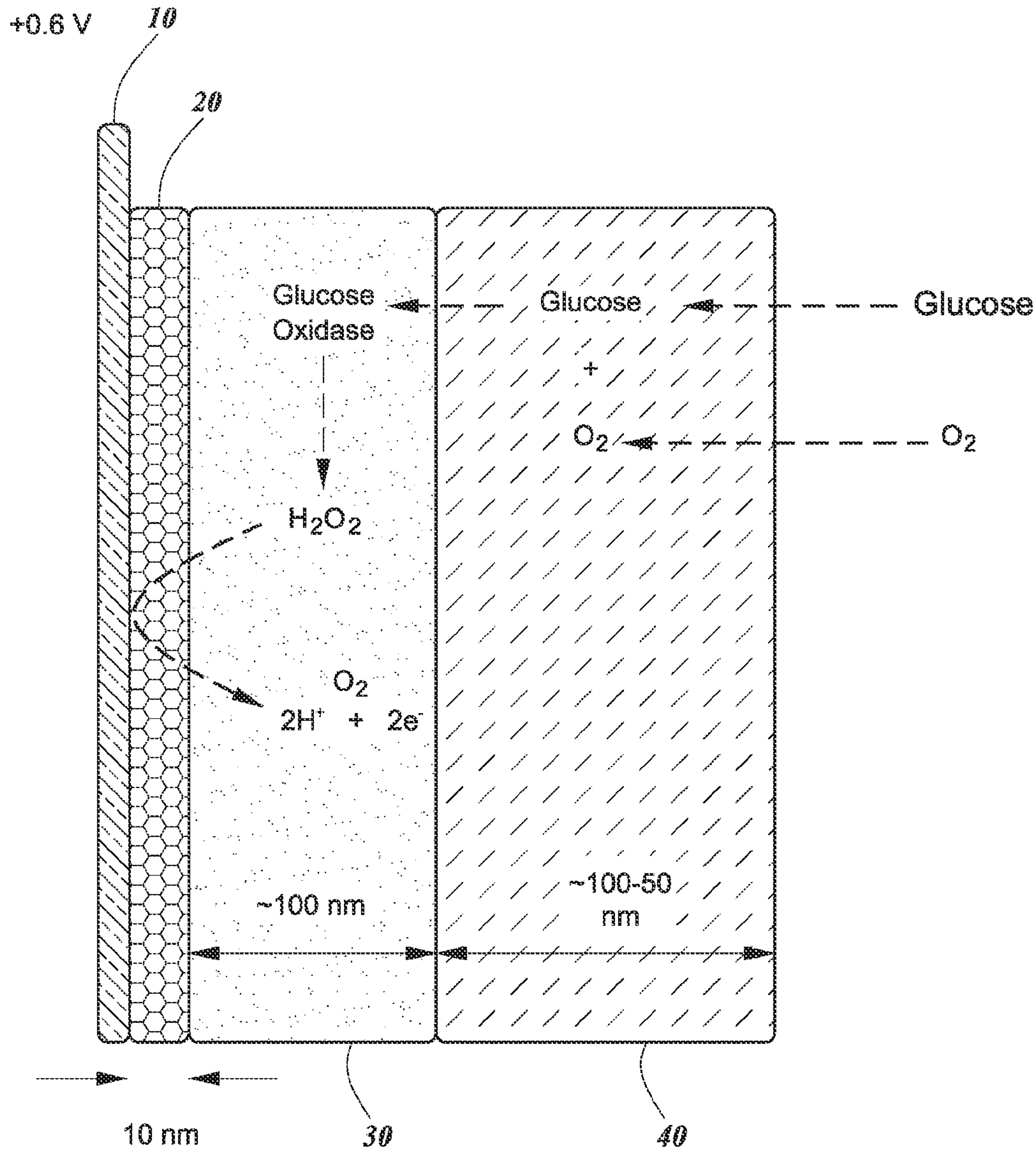
+0.6 V
10
20
Glucose
Oxidase
Glucose
Glucose
+
$O_2$
$O_2$
$H_2O_2$
$O_2$
$2H^+$ + $2e^-$
~100 nm
~100-50
nm
10 nm
30
40

FIG. 1A

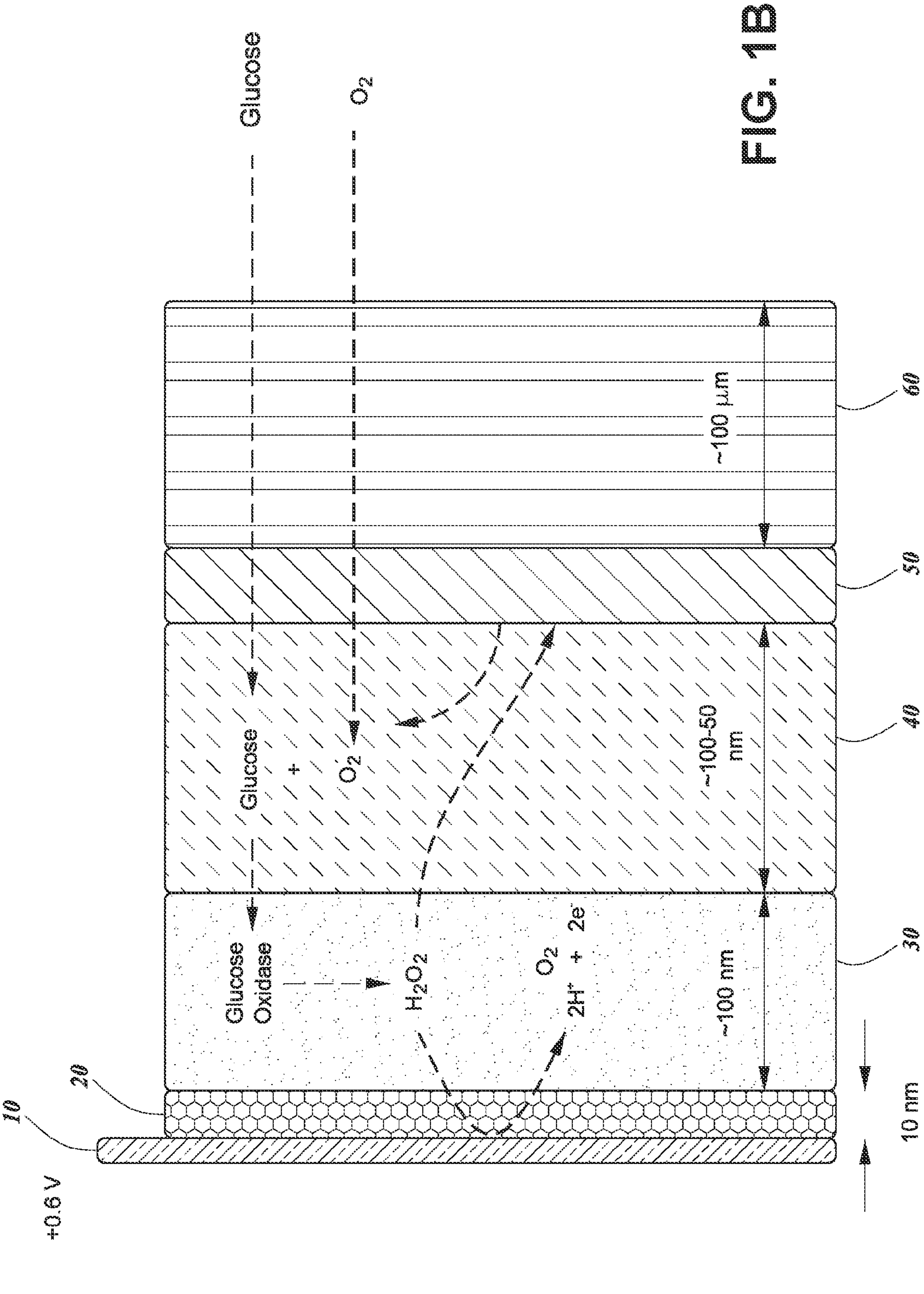

Glucose
O2
Glucose
+
O2
Glucose
Oxidase
H2O2
O2
2H+ + 2e-
~100 μm
~100-50
nm
~100 nm
10 nm
+0.6 V
10
20
30
40
50
60


FIG. 1B

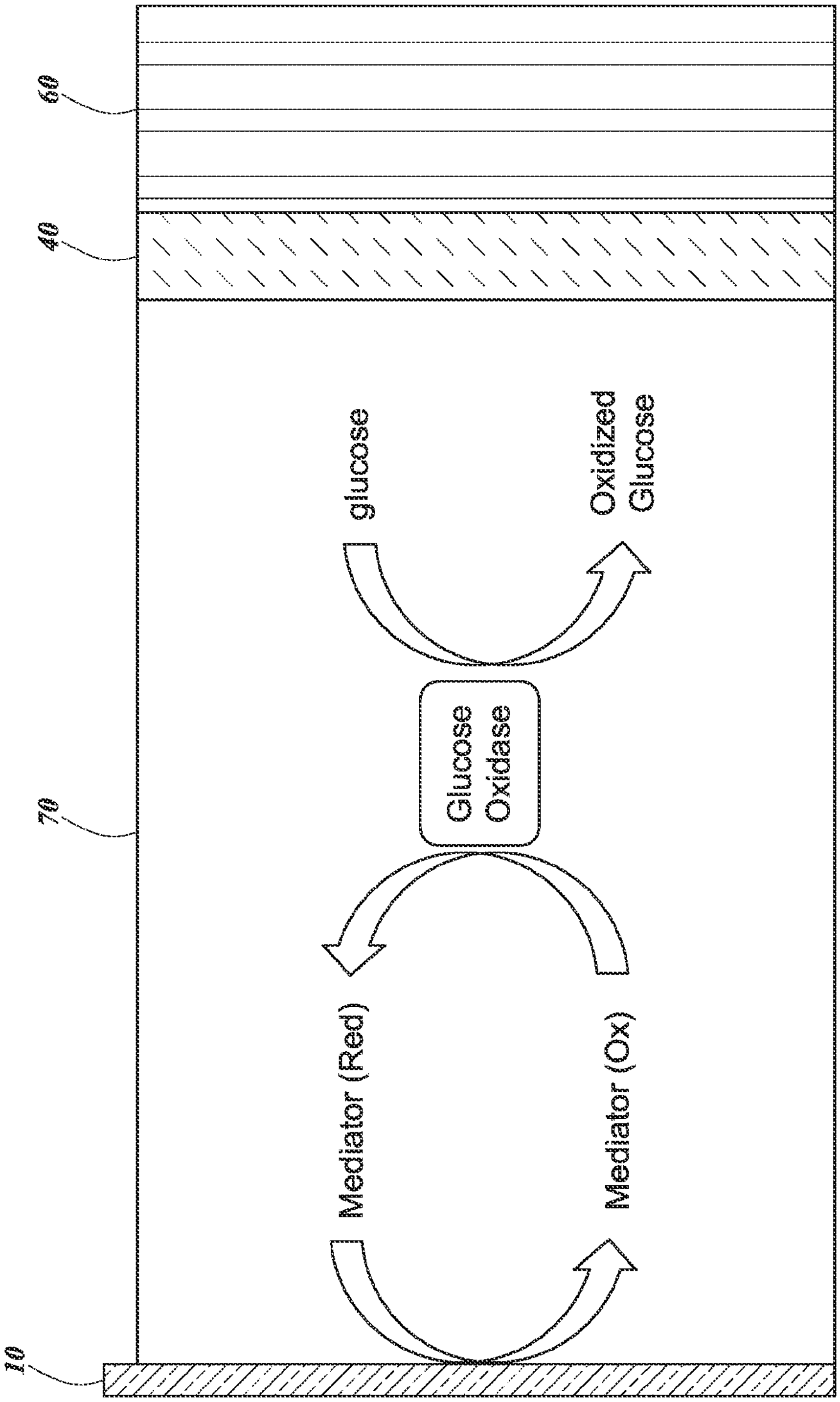
60
40
70
10
glucose
Oxidized Glucose
Glucose Oxidase
Mediator (Red)
Mediator (Ox)

FIG. 1C

FIG. 2

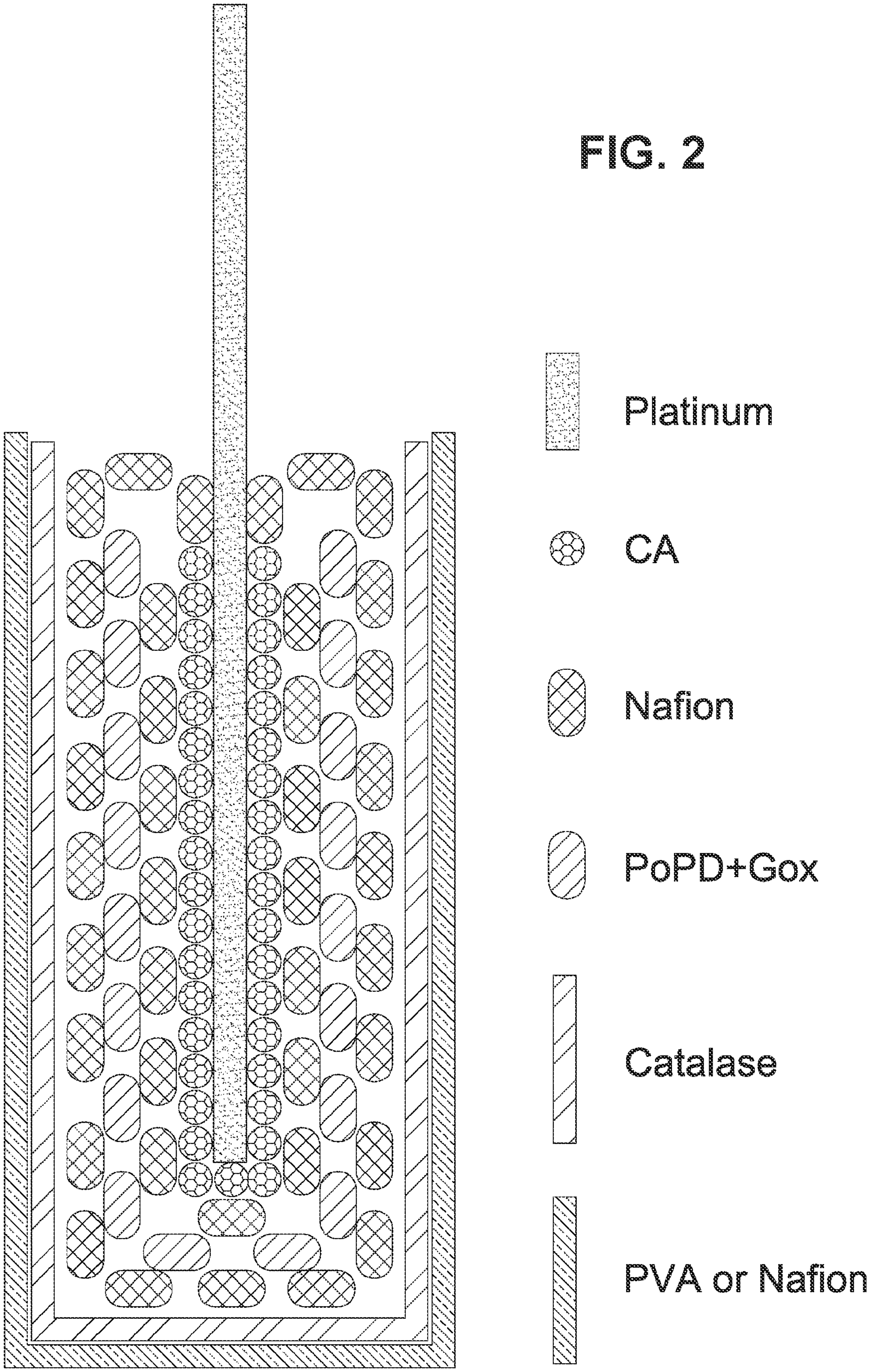

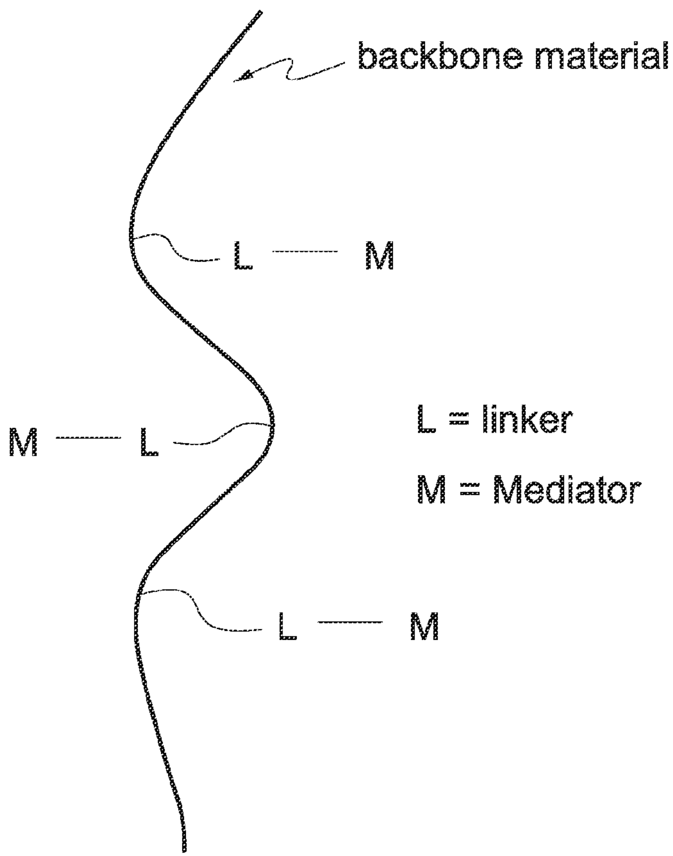
backbone material
L —— M
M —— L
L = linker
M = Mediator
L —— M

FIG. 3A

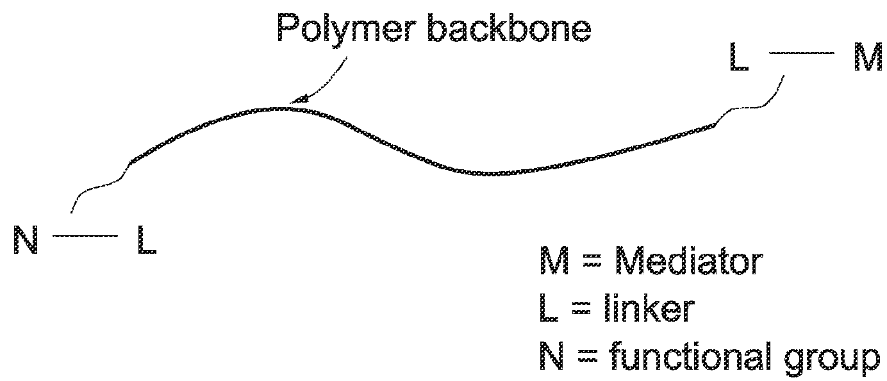
Polymer backbone
L —— M
N —— L
M = Mediator
L = linker
N = functional group

FIG. 3B

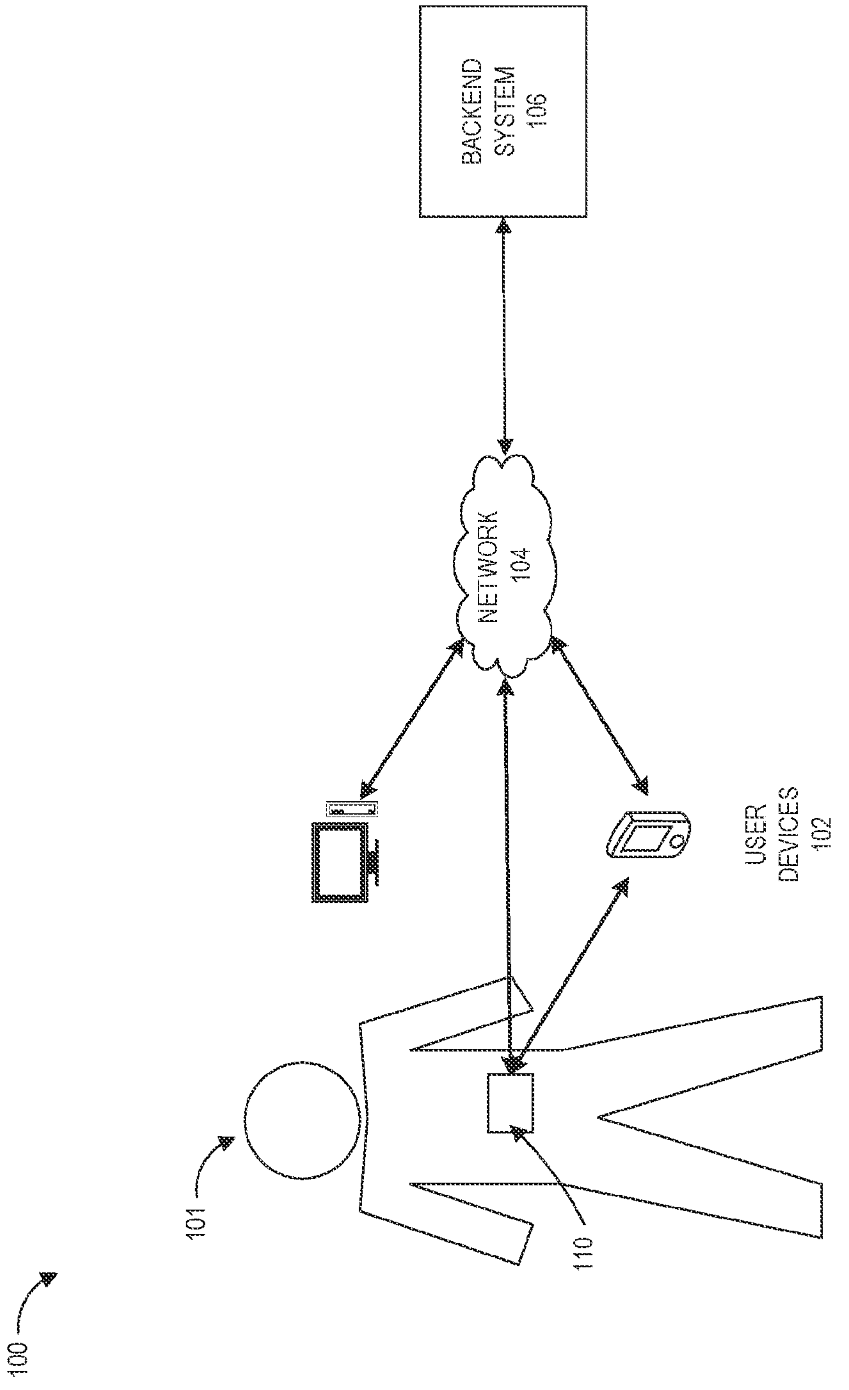


FIG. 4A

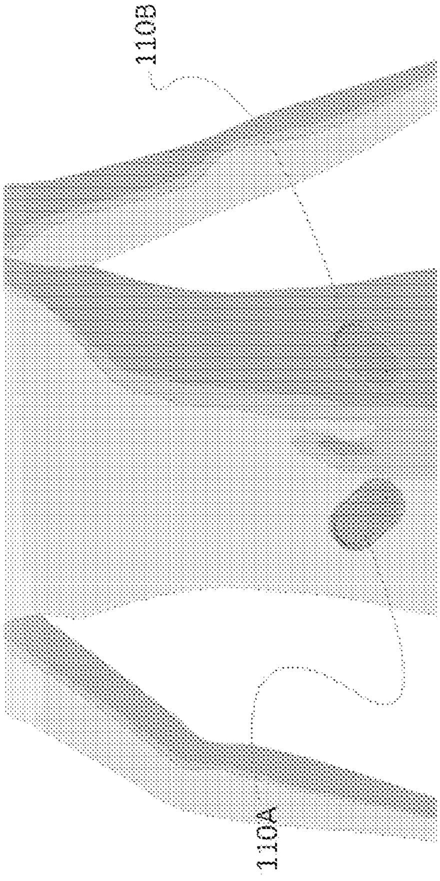


FIG. 4B

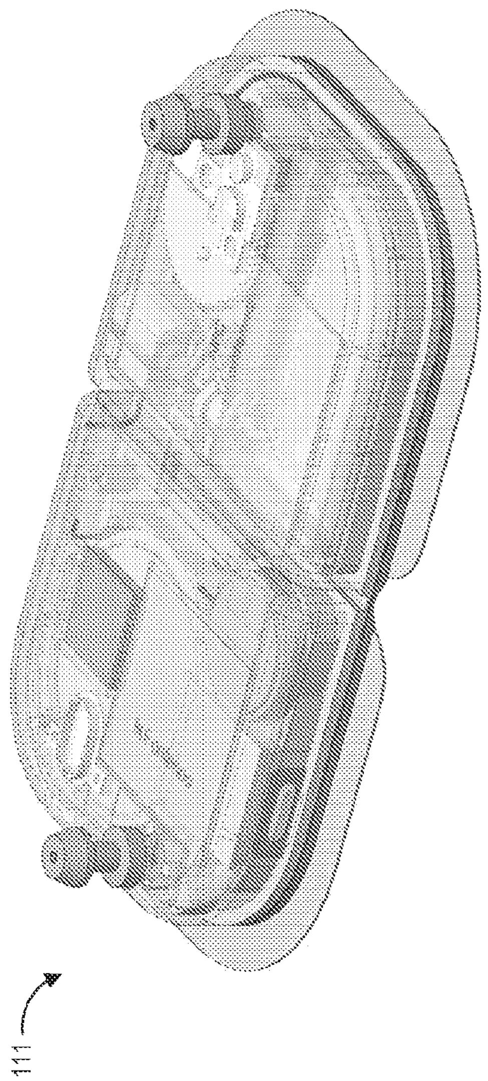


FIG. 4C

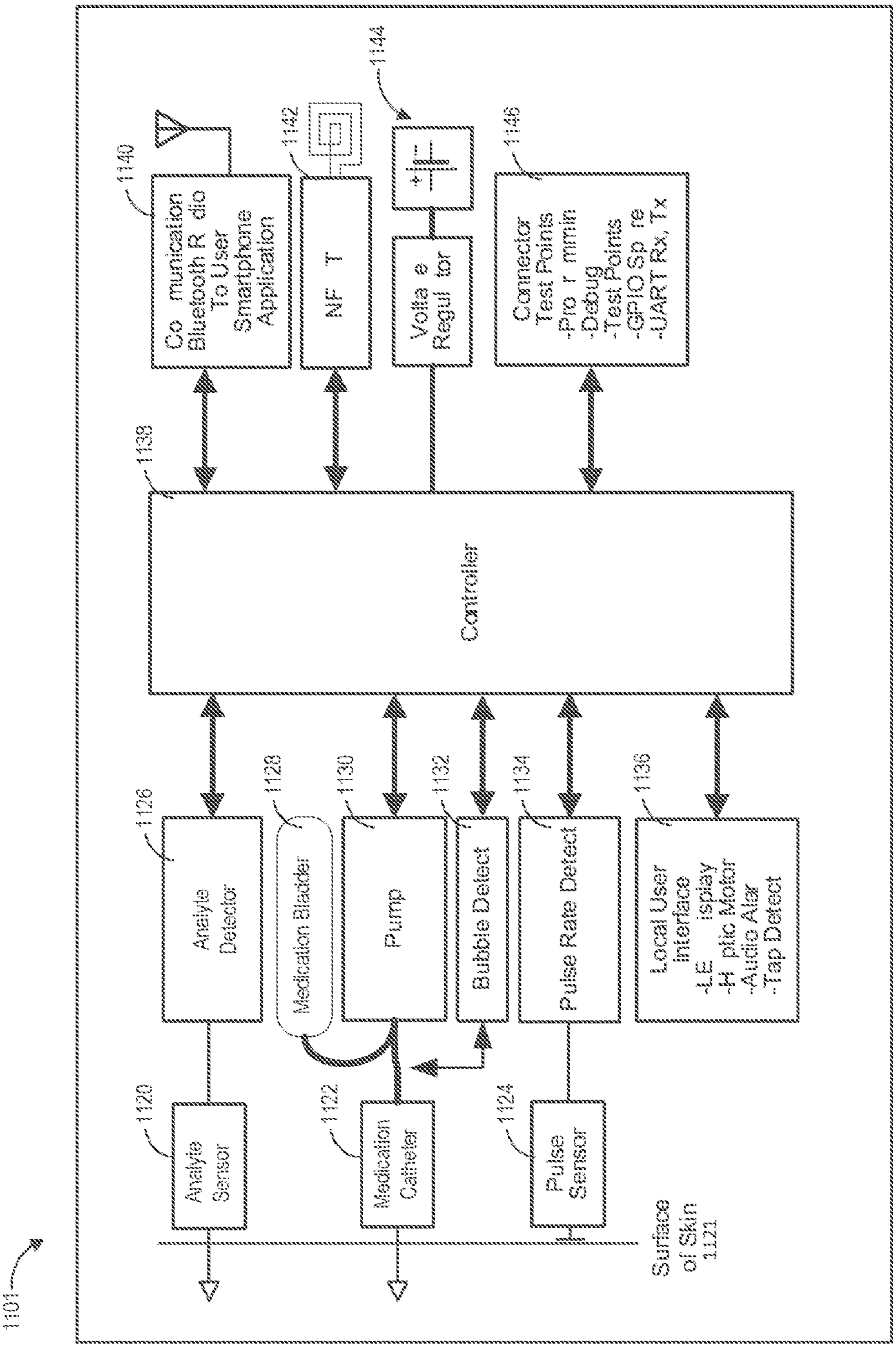
1101
1120
Analyte Sensor
1122
Medication Catheter
1124
Pulse Sensor
Surface of Skin 1121
1126
Analyte Detector
1128
Medication Bladder
1130
Pump
1132
Bubble Detect
1134
Pulse Rate Detect
1136
Local User Interface
-LE isplay
-H ptic Motor
-Audio Alar
-Tap Detect
1138
Controller
1140
Co munication
Bluetooth R dio
To User
Smartphone
Application
1142
NF T
1144
Volta e
Regul tor
1146
Connector
Test Points
-Pro r mmin
-Debug
-Test Points
-GPIO Sp re
-UART Rx, Tx

FIG. 5

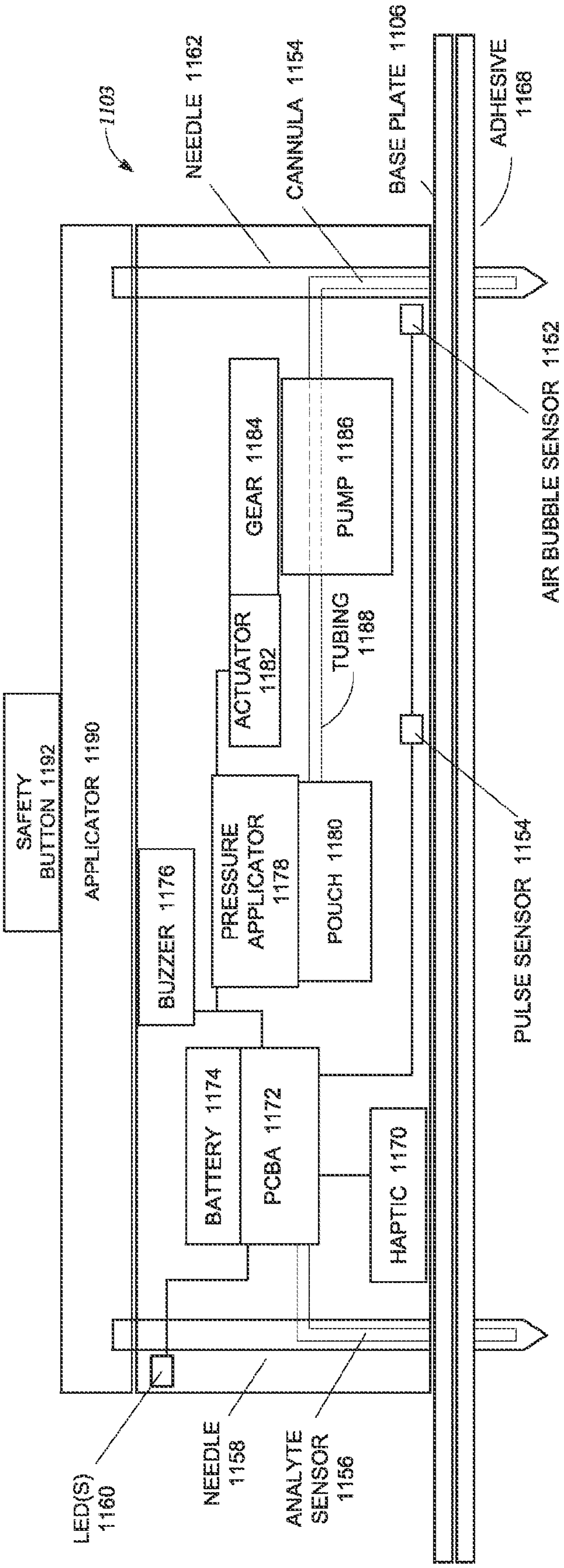
1103
NEEDLE 1162
CANNULA 1154
BASE PLATE 1106
ADHESIVE 1168
SAFETY BUTTON 1192
APPLICATOR 1190
BUZZER 1176
PRESSURE APPLICATOR 1178
POUCH 1180
ACTUATOR 1182
GEAR 1184
PUMP 1186
TUBING 1188
AIR BUBBLE SENSOR 1152
PULSE SENSOR 1154
BATTERY 1174
PCBA 1172
HAPTIC 1170
LED(S) 1160
NEEDLE 1158
ANALYTE SENSOR 1156

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5431160 A
- US 2017238851 A1
- US 20210236729 A